# EUROPEAN PATENT SPECIFICATION

(11) **EP 4 426 422 B1**
(45) Date of publication and mention of the grant of the patent: **24.12.2025**
(21) Application number: 22823185.8
(22) Date of filing: 04.11.2022
(51) Int. Cl.: A61N 1/372, A61N 1/36, A61N 1/05

(54) **PRESENTATION OF ELECTROSTIMULATION AND CLINICAL RESPONSE DATA**
DARSTELLUNG VON ELEKTROSTIMULATIONS- UND KLINISCHEN REAKTIONSDATEN
PRÉSENTATION DE DONNÉES D'ÉLECTROSTIMULATION ET DE RÉPONSE CLINIQUE

(30) Priority: 05.11.2021 US 202163276027 P
(43) Date of publication of application: 11.09.2024
(73) Proprietor: Boston Scientific Neuromodulation Corporation, Valencia, CA 91355 (US)
(72) Inventor: MOORE, Lisa Denise, Glendale, CA 91214 (US); FISCHELL, Rachel Mae, West Hills, CA 91307 (US)
(74) Representative: Vossius & Partner Patentanwälte Rechtsanwälte mbB
(86) International application number: PCT/US2022/048962
(87) International publication number: WO 2023/081357

(56) References cited:
- US-A1- 2019 134 403
- US-A1- 2020 155 859
- US-A1- 2021 196 956
- US-B2- 9 492 673

## Description

### TECHNICAL FIELD

This document relates generally to medical devices, and more particularly, to systems and devices for presenting electrostimulation data and patient clinical responses to electrostimulation in a monopolar review format.

### BACKGROUND

Neuromodulation (or "neural neuromodulation", also referred to as "neurostimulation" or "neural stimulation") has been proposed as a therapy for a number of conditions. Often, neuromodulation and neural stimulation may be used interchangeably to describe excitatory stimulation that causes action potentials as well as inhibitory and other effects. Examples of neuromodulation include Spinal Cord Stimulation (SCS), Deep Brain Stimulation (DBS), Peripheral Nerve Stimulation (PNS), and Functional Electrical Stimulation (FES). SCS systems have been used as a therapeutic modality for the treatment of chronic pain syndromes. PNS has been used to treat chronic pain syndrome and incontinence, with a number of other applications under investigation. FES systems have been applied to restore some functionality to paralyzed extremities in spinal cord injury patients. DBS can be used to treat a variety of diseases or disorders.

Stimulation systems, such as implantable electrostimulators, have been developed to provide therapy for a variety of treatments. An implantable electrostimulator can include a pulse generator and one or more leads each including a plurality of stimulation electrodes. The stimulation electrodes are in contact with or near target tissue to be stimulated, such as nerves, muscles, or other tissue. The control module generates a control signal to the pulse generator, which generates electrostimulation pulses that are delivered by the electrodes to the target tissue in accordance with an electrode configuration and a set of stimulation parameters.

US2020/155859 discloses a system for providing electrostimulation to a patient comprising an implantable stimulator and a programming device.

### SUMMARY

The invention is defined by independent claim 1. Various examples discussed in this document may provide more effective presentation of stimulation parameter space (e.g., electrode configurations and parameter values) and patient clinical response data associated with tested or untested stimulation settings. In accordance with various examples discussed in this document, tested clinical response data corresponding to a first set of base stimulation settings and predicted clinical response data corresponding to a different second set of base stimulation settings can be generated. Unlike the tested clinical response data that are based on electrostimulation actually delivered to the patient in accordance with a base stimulation setting of the first set, the predicted clinical response data can be generated without delivering electrostimulation but based on a prediction model. The tested and predicted clinical response data can be used to determine, for one or more monopolar electrode configurations, characteristic stimulation amplitudes with respective clinical response data satisfying respective conditions. The characteristic stimulation amplitudes for each of a range of electrodes on the lead, optionally along with the actual ad predicted clinical response data of the first and second sets of base stimulation settings, can be formatted and presented to a system user (e.g., a physician).

The formatted report as described herein is a comprehensive and effective presentation of the electrode and stimulation parameter search space. It can help physicians better understand clinical effects of electrostimulations delivered via a wide range of tested and untested electrode configurations and stimulation parameter values or value ranges. Monopolar reviews can be sensitive and specific to movement disorders such as tremor, rigidity, or bradykinesia. The formatted data report also facilitates consistent data reporting and data entry into other systems such as patient medical record system, thereby easing the human workload and saving the cost of entry of such stimulation data. The formatted data report can also improve communication between physicians on therapy programming results, and increase understanding and usability of advanced algorithms such as automated search for base stimulation settings and prediction of clinical effects for untested stimulation settings, and devices running such algorithms.

The description that follows will generally focus on the use of the invention within a DBS system, such as that disclosed in U.S. Patent Application Publication 2020/0001091. However, the present invention may find applicability with any implantable neurostimulator device system, including Spinal Cord Stimulation (SCS) systems, Vagus Nerve Stimulation (VNS) system, Sacral Nerve Stimulation (SNS) systems, and the like.

This Summary is an overview of some of the teachings of the present application and not intended to be an exclusive or exhaustive treatment of the present subject matter. Further details about the present subject matter are found in the detailed description and appended claims. Other aspects of the disclosure will be apparent to persons skilled in the art upon reading and understanding the following detailed description and viewing the drawings that form a part thereof, each of which are not to be taken in a limiting sense. The scope of the present disclosure is defined by the appended claims.

### BRIEF DESCRIPTION OF THE DRAWINGS

Various examples are illustrated by way of example in the figures of the accompanying drawings. Such examples are demonstrative and not intended to be exhaustive or exclusive examples of the present subject matter.
FIG. 1 illustrates, by way of example and not limitation, an electrical stimulation system that may be used to deliver deep brain stimulation (DBS).
FIG. 2 illustrates, by way of example and not limitation, an implantable pulse generator (IPG) that may be used in a DBS system.
FIGS. 3A-3B illustrate, by way of example and not limitation, leads that may be coupled to an IPG to deliver electrostimulation such as DBS.
FIG. 4 illustrates, by way of example and not limitation, a computing device for programming or controlling the operation of an electrostimulation system.
FIG. 5 illustrates, by way of example and not limitation, a stimulation parameter control system and a part of the environment in which it may operate.
FIG. 6 illustrates, by way of example and not limitation, a graphical user interface (GUI) for programming a stimulation setting into an IPG.
FIG. 7 illustrates, by way of example and not limitation, a diagram of an electrode configuration and parameter search space and a two-dimensional (2D) monopolar review of characteristic stimulation amplitudes corresponding to ring mode stimulation.
FIG. 8 illustrates, by way of example and not limitation, a diagram of an electrode configuration and parameter search space and a 2D monopolar review monopolar review of characteristic stimulation amplitudes corresponding to directional mode stimulation.
FIG. 9 is a flow chart illustrating, by way of example and not limitation, a method for generating and presenting electrostimulation data and actual and predicted patient clinical responses in a formatted report to guide electrostimulation therapy.
FIG. 10 illustrates generally a block diagram of an example machine upon which any one or more of the techniques (e.g., methodologies) discussed herein may perform.

### DETAILED DESCRIPTION

This document describes systems and non-claimed methods for presenting electrostimulation data and patient clinical responses to electrostimulation. According to an example, a system comprises an implantable stimulator, and a programming device including a controller to identify first and second sets of base stimulation settings each comprising an electrode configuration and stimulation parameter values selected from a configuration and parameter search space. The controller can detect clinical effects and evaluate a clinical response indicator in response to electrostimulation for each base stimulation setting of the first set, and predict clinical effects and estimate a clinical response indicator without delivering electrostimulation for each base stimulation setting of the second set. Based on the clinical response indicators, the controller can determine characteristic stimulation amplitudes for one or more electrode configurations. A formatted monopolar review report comprising the characteristic stimulation amplitudes can be displayed to the user to assist programming of a electrostimulation therapy.

Various examples described herein involve deep brain stimulation (DBS). The following detailed description of the present subject matter refers to the accompanying drawings which show, by way of illustration, specific aspects and examples in which the present subject matter may be practiced. These examples are described in sufficient detail to enable those skilled in the art to practice the present subject matter. Other examples may be utilized and structural, logical, and electrical changes may be made without departing from the scope of the present subject matter. References to "an", "one", or "various" examples in this disclosure are not necessarily to the same example, and such references contemplate more than one example. The following detailed description is, therefore, not to be taken in a limiting sense, and the scope is defined only by the appended claims, along with the full scope of legal equivalents to which such claims are entitled.

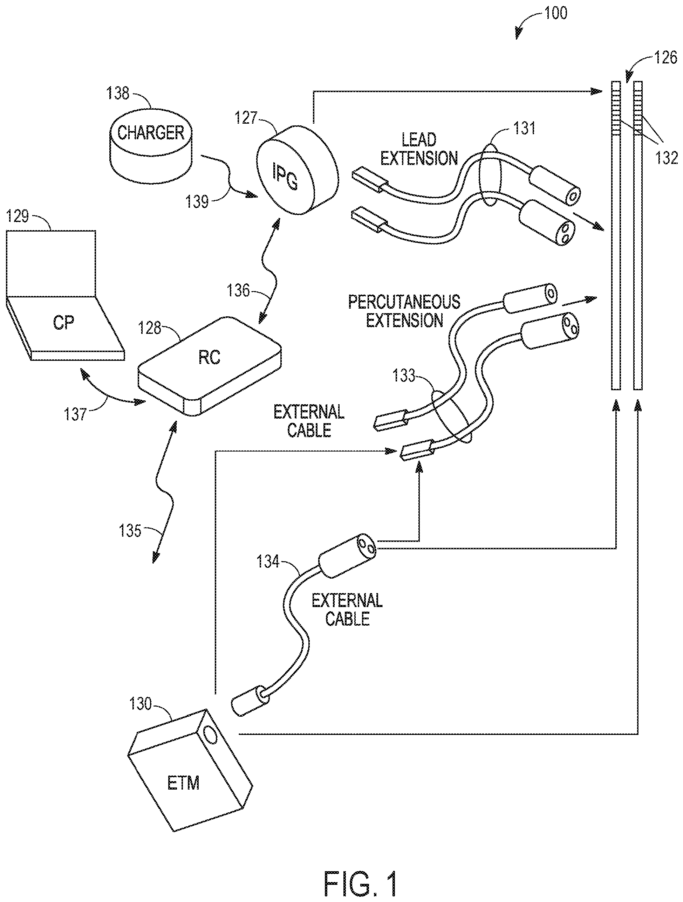

FIG. 1 illustrates, by way of example, an example of an electrical stimulation system 100, which may be used to deliver DBS. The electrical stimulation system 100 may generally include a one or more (illustrated as two) of implantable neuromodulation leads 126, an implantable pulse generator (IPG) 127, an external remote controller (RC) 128, a clinician programmer (CP) 129, and an external trial modulator (ETM) 130. The IPG 127 may be physically connected via one or more percutaneous lead extensions 131 to the neuromodulation lead(s) 126, which carry a plurality of electrodes 132. The electrodes, when implanted in a patient, form an electrode arrangement. As illustrated, the neuromodulation leads 126 may be percutaneous leads with the electrodes arranged in-line along the neuromodulation leads or about a circumference of the neuromodulation leads. Any suitable number of neuromodulation leads can be provided, including only one, as long as the number of electrodes is greater than two (including the IPG case function as a case electrode) to allow for lateral steering of the current. Alternatively, a surgical paddle lead can be used in place of one or more of the percutaneous leads. The IPG 127 includes pulse generation circuitry that delivers electrical modulation energy in the form of a pulsed electrical waveform (*i.e.,* a temporal series of electrical pulses) to the electrodes in accordance with a set of modulation parameters.

The ETM 130 may also be physically connected via the percutaneous lead extensions 133 and external cable 134 to the neuromodulation lead(s) 126. The ETM 130 may have similar pulse generation circuitry as the IPG 127 to deliver electrical modulation energy to the electrodes in accordance with a set of modulation parameters. The ETM 130 is a non-implantable device that may be used on a trial basis after the neuromodulation leads 126 have been implanted and prior to implantation of the IPG 127, to test the responsiveness of the modulation that is to be provided. Functions described herein with respect to the IPG 127 can likewise be performed with respect to the ETM 130.

The RC 128 may be used to telemetrically control the ETM 130 via a bi-directional RF communications link 135. The RC 128 may be used to telemetrically control the IPG 127 via a bi-directional RF communications link 136. Such control allows the IPG 127 to be turned on or off and to be programmed with different modulation parameter sets. The IPG 127 may also be operated to modify the programmed modulation parameters to actively control the characteristics of the electrical modulation energy output by the IPG 127. A clinician may use the CP 129 to program modulation parameters into the IPG 127 and ETM 130 in the operating room and in follow-up sessions.

The CP 129 may indirectly communicate with the IPG 127 or ETM 130, through the RC 128, via an IR communications link 137 or another link. The CP 129 may directly communicate with the IPG 127 or ETM 130 via an RF communications link or other link (not shown). The clinician detailed modulation parameters provided by the CP 129 may also be used to program the RC 128, so that the modulation parameters can be subsequently modified by operation of the RC 128 in a stand-alone mode (i.e., without the assistance of the CP 129). Various devices may function as the CP 129. Such devices may include portable devices such as a lap-top personal computer, mini-computer, personal digital assistant (PDA), tablets, phones, or a remote control (RC) with expanded functionality. Thus, the programming methodologies can be performed by executing software instructions contained within the CP 129. Alternatively, such programming methodologies can be performed using firmware or hardware. In any event, the CP 129 may actively control the characteristics of the electrical modulation generated by the IPG 127 to allow the desired parameters to be determined based on patient feedback or other feedback and for subsequently programming the IPG 127 with the desired modulation parameters. To allow the user to perform these functions, the CP 129 may include user input device (e.g., a mouse and a keyboard), and a programming display screen housed in a case. In addition to, or in lieu of, the mouse, other directional programming devices may be used, such as a trackball, touchpad, joystick, touch screens or directional keys included as part of the keys associated with the keyboard. An external device (e.g. CP) may be programmed to provide display screen(s) that allow the clinician to, among other functions, select or enter patient profile information (e.g., name, birth date, patient identification, physician, diagnosis, and address), enter procedure information (e.g., programming/follow-up, implant trial system, implant IPG, implant IPG and lead(s), replace IPG, replace IPG and leads, replace or revise leads, explant, etc.), generate a pain map of the patient, define the configuration and orientation of the leads, initiate and control the electrical modulation energy output by the neuromodulation leads, and select and program the IPG with modulation parameters, including electrode selection, in both a surgical setting and a clinical setting. The display screen(s) may be used to suggest the electrode(s) for use to stimulate a targeted dorsal root. The external device(s) (e.g. CP and/or RC) may be configured to communicate with other device(s), including local device(s) and/or remote device(s). For example, wired and/or wireless communication may be used to communicate between or among the devices.

An external charger 138 may be a portable device used to transcutaneous charge the IPG 127 via a wireless link such as an inductive link 139. Once the IPG 127 has been programmed, and its power source has been charged by the external charger or otherwise replenished, the IPG 127 may function as programmed without the RC 128 or CP 129 being present.

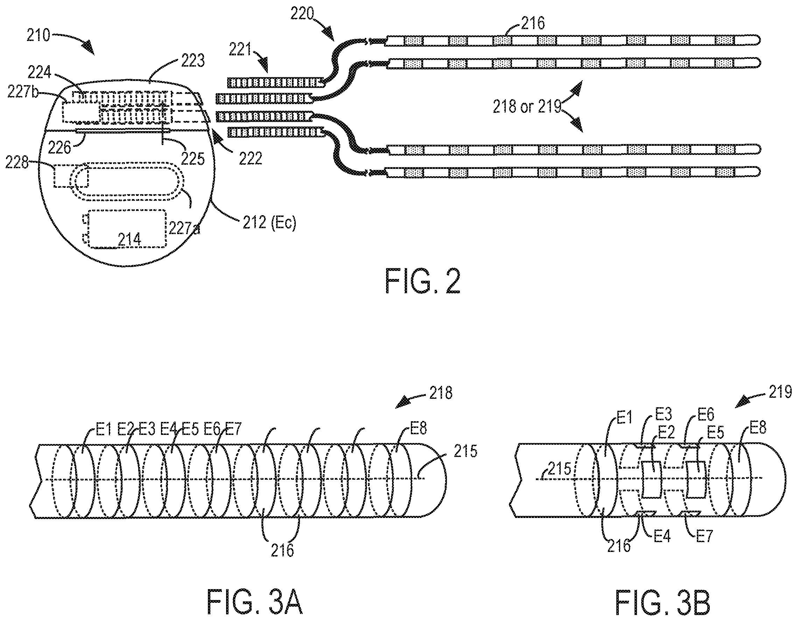

FIG. 2 illustrates, by way of example and not limitation, an IPG 210 in a DBS system. The IPG 210, which is an example of the IPG 127 of the electrical stimulation system 100 as illustrated in FIG. 1, may include a biocompatible device case 212 that holds the circuitry and a battery 214 for providing power for the IPG 210 to function, although the IPG 210 can also lack a battery and can be wirelessly powered by an external source. The IPG 210 may be coupled to one or more leads, such as leads 218 or 219 as illustrated herein. The lead 218 or 219 can each include a plurality of electrodes 216 for delivering electrostimulation energy, recording electrical signals, or both. In some examples, the leads 218 or 219 can be rotatable so that the electrodes 216 can be aligned with the target neurons after the neurons have been located such as based on the recorded signals.

The leads 218 or 219 can be implanted near or within the desired portion of the body to be stimulated. In an example of operations for DBS, access to the desired position in the brain can be accomplished by drilling a hole in the patient's skull or cranium with a cranial drill (commonly referred to as a burr), and coagulating and incising the dura mater, or brain covering. A lead can then be inserted into the cranium and brain tissue with the assistance of a stylet (not shown). The lead can be guided to the target location within the brain using, for example, a stereotactic frame and a microdrive motor system. In some examples, the microdrive motor system can be fully or partially automatic. The microdrive motor system may be configured to perform actions such as inserting, advancing, rotating, or retracing the lead.

Lead body of the leads 218 or 219 can be formed of a biocompatible, non-conducting material such as, for example, a polymeric material. Suitable polymeric materials include, but are not limited to, silicone, polyurethane, polyurea, polyurethane-urea, polyethylene, or the like. Once implanted in the body, the leads 218 or 219 may be in contact with body tissue for extended periods of time. In some examples, the leads 218 or 219 can have a cross-sectional diameter of no more than 1.5 mm and may be in the range of 0.5 to 1.5 mm.

The electrodes 216 can be made of metal, alloy, conductive oxide, or any other suitable conductive biocompatible material. Examples of suitable materials include, but are not limited to, platinum, platinum iridium alloy, iridium, titanium, tungsten, palladium, palladium rhodium, or the like. Preferably, the electrodes are made of a material that is biocompatible and does not substantially corrode under expected operating conditions in the operating environment for the expected duration of use. The electrodes 216 can include one or more ring electrodes, and/or one or more sets of segmented electrodes (or any other combination of electrodes), examples of which are discussed below with reference to FIGS. 3A and 3B.

Lead wires 220 within the leads may be coupled to the electrodes 216 and to proximal contacts 221 insertable into lead connectors 222 fixed in a header 223 on the IPG 210, which header can comprise an epoxy for example. Alternatively, the proximal contacts 221 may connect to lead extensions (not shown) which are in turn inserted into the lead connectors 222. Once inserted, the proximal contacts 221 connect to header contacts 224 within the lead connectors 222, which are in turn coupled by feedthrough pins 225 through a case feedthrough 226 to stimulation circuitry 228 within the case 212, which stimulation circuitry 228 is described below.

By way of example and not limitation, the IPG 210 illustrated in FIG. 2 can be coupled to four percutaneous leads 218 or 219 (218 is shown), and thus the header 223 may include a 2x2 array of eight-electrode lead connectors 222. However, the type and number of leads, and the number of electrodes, in an IPG is application specific and therefore can vary. In another example not shown, a given lead can have sixteen electrodes, and thus this lead would have two sets of proximal contacts 221 to mate with two of the eight-electrode lead connectors 222, as disclosed for example in U.S. Patent Application Publication 2019/0076645. The conductive case 212 can also comprise an electrode (Ec).

In a DBS application, as is useful in the treatment of tremor in Parkinson's disease for example, the IPG 210 is typically implanted under the patient's clavicle (collarbone). The leads 218 or 219 (which may be extended by lead extensions, not shown) can be tunneled through and under the neck and the scalp, with the electrodes 216 implanted through holes drilled in the skull and positioned for example in the subthalamic nucleus (STN) and the pedunculopontine nucleus (PPN) in each brain hemisphere. The IPG 210 can also be implanted underneath the scalp closer to the location of the electrodes' implantation. The leads 218 or 219, or the extensions 633, can be integrated with and permanently connected to the IPG 210 in other solutions.

The IPG 210 can include an antenna 227a allowing it to communicate bi-directionally with a number of external devices discussed subsequently. The antenna 227a as shown comprises a conductive coil within the case 212, although the coil of the antenna 227a can also appear in the header 223. When the antenna 227a is configured as a coil, communication with external devices preferably occurs using near-field magnetic induction. The IPG 210 may also include a Radio-Frequency (RF) antenna 227b. Although the RF antenna 227b is shown within the header 223, in some examples it may also be within the case 212. The RF antenna 227b may comprise a patch, slot, or wire, and may operate as a monopole or dipole. The RF antenna 227b preferably communicates using far-field electromagnetic waves, and may operate in accordance with any number of known RF communication standards, such as Bluetooth, Zigbee, WiFi, MICS, and the like. If the IPG 210 lacks a battery 214, an additional coil can be present to receive wireless power from an external source.

Stimulation in IPG 210 is typically provided by pulses each of which may include one phase or multiple phases. For example, a monopolar stimulation current can be delivered between a lead-based electrode (e.g., one of the electrodes 216) and the case electrode Ec 212. A bipolar stimulation current can be delivered between two lead-based electrodes (e.g., two of the electrodes 216). Stimulation parameters typically include current amplitude (or voltage amplitude), frequency, pulse width of the pulses or of its individual phases; electrodes selected to provide the stimulation; polarity of such selected electrodes, i.e., whether they act as anodes that source current to the tissue, or cathodes that sink current from the tissue. Each of the electrodes can either be used (an active electrode) or unused (OFF). When the electrode is used, the electrode can be used as an anode or cathode and carry anodic or cathodic current. In some instances, an electrode might be an anode for a period of time and a cathode for a period of time. These and possibly other stimulation parameters taken together comprise a stimulation program that the stimulation circuitry 228 in the IPG 210 can execute to provide therapeutic stimulation to a patient.

In some examples, a measurement device coupled to the muscles or other tissue stimulated by the target neurons, or a unit responsive to the patient or clinician, can be coupled to the IPG 210 or microdrive motor system. The measurement device, user, or clinician can indicate a response by the target muscles or other tissue to the stimulation or recording electrode(s) to further identify the target neurons and facilitate positioning of the stimulation electrode(s). For example, if the target neurons are directed to a muscle experiencing tremors, a measurement device can be used to observe the muscle and indicate changes in, for example, tremor frequency or amplitude in response to stimulation of neurons. Alternatively, the patient or clinician can observe the muscle and provide feedback.

FIGS. 3A-3B illustrate, by way of example and not limitation, leads 216 and 218 that may be coupled to the IPG 210 to deliver electrostimulation such as DBS. FIG. 3A shows a lead 218 with electrodes 216 disposed at least partially about a circumference of the lead 218. The electrodes 216 may be located along a distal end portion of the lead 218. As illustrated herein, the electrodes 216 are ring electrodes that span 360 degrees about a circumference of the lead 218. A ring electrode allows current to project equally in every direction from the position of the electrode, and typically does not enable stimulus current to be directed from only a particular angular position or a limited angular range around of the lead. The lead 218, which includes only ring electrodes, is also referred to as a non-directional lead.

FIG. 3B shows a lead 219 with electrodes 216 including ring electrodes such as E1 at a proximal end and E8 at the distal end. Additionally, the lead 219 also include a plurality of segmented electrodes (also known as split-ring electrodes). For example, a set of segmented electrodes E2, E3, and E4 are around the circumference at a longitudinal position, each spanning less than 360 degrees around the lead axis 215. In an example, each of electrodes E2, E3, and E4 spans 90 degrees, with each being separated from the others by gaps of 30 degrees. Another set of segmented electrodes E5, E6, and E7 are located around the circumference at another longitudinal position different from the segmented electrodes E2, E3 and E4. Segmented electrodes such as E2-E7 can direct stimulus current to a selected angular range around the lead.

Segmented electrodes can typically provide superior current steering than ring electrodes because target structures in DBS or other stimulation are not typically symmetric about the axis of the distal electrode array. Instead, a target may be located on one side of a plane running through the axis of the lead. Through the use of a radially segmented electrode array, current steering can be performed not only along a length of the lead but also around a circumference of the lead. This provides precise three-dimensional targeting and delivery of the current stimulus to neural target tissue, while potentially avoiding stimulation of other tissue. In some examples, segmented electrodes can be together with ring electrodes. The lead 219, which include at least one or more segmented electrodes, is also referred to as a directional lead. In an example, all electrodes on a directional lead can be segmented electrodes. In another example, there can be different numbers of segmented electrodes at different longitudinal positions.

As illustrated in FIG. 3B, segmented electrodes may be grouped into sets of segmented electrodes, where each set is disposed around a circumference at a particular longitudinal location of the directional lead 219. The directional lead 219 may have any number (e.g., three as shown in FIG. 3B) segmented electrodes in a given set of segmented electrodes. By way of example and not limitation, a given set may include any number between two to 16 segmented electrodes. In an example, all sets of segmented electrodes may contain the same number of segmented electrodes. In another example, one set of the segmented electrodes may include a different number of electrodes than at least one other set of segmented electrodes.

The segmented electrodes may vary in size and shape. In some examples, the segmented electrodes are all of the same size, shape, diameter, width or area or any combination thereof. In some examples, the segmented electrodes of each circumferential set (or even all segmented electrodes disposed on the lead 219) may be identical in size and shape.

Each set of segmented electrodes may be disposed around the circumference of the lead body to form a substantially cylindrical shape around the lead body. The spacing between individual electrodes of a given set of the segmented electrodes may be the same, or different from, the spacing between individual electrodes of another set of segmented electrodes on the lead 219. In some examples, equal spaces, gaps or cutouts are disposed between each segmented electrode around the circumference of the lead 219. In other examples, the spaces, gaps or cutouts between the segmented electrodes may differ in size or shape. In other examples, the spaces, gaps, or cutouts between segmented electrodes may be uniform for a particular set of the segmented electrodes, or for all sets of the segmented electrodes. The sets of segmented electrodes may be positioned in irregular or regular intervals along a length the lead 219.

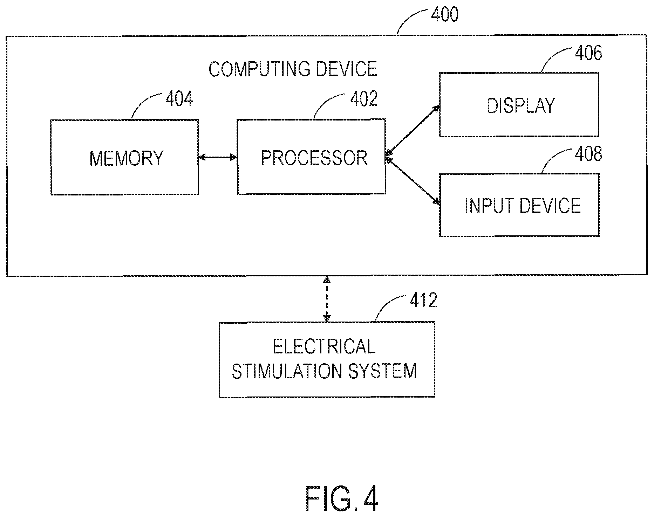

FIG. 4 illustrates, by way of example and not limitation, a computing device 400 for programming or controlling the operation of an electrical stimulation system 412. The computing device 400 includes a processor 402, a memory 404, a display 406, and an input device 408. Optionally, the computing device 400 may be separate from and communicatively coupled to the electrical stimulation system 412. Alternatively, the computing device 400 may be integrated with the electrical stimulation system 412. In an example, the computing device 400 can be a part of the electrical stimulation system 412, such as part of the IPG 127, RC 128, CP 129, or ETM 130 illustrated in FIG. 1.

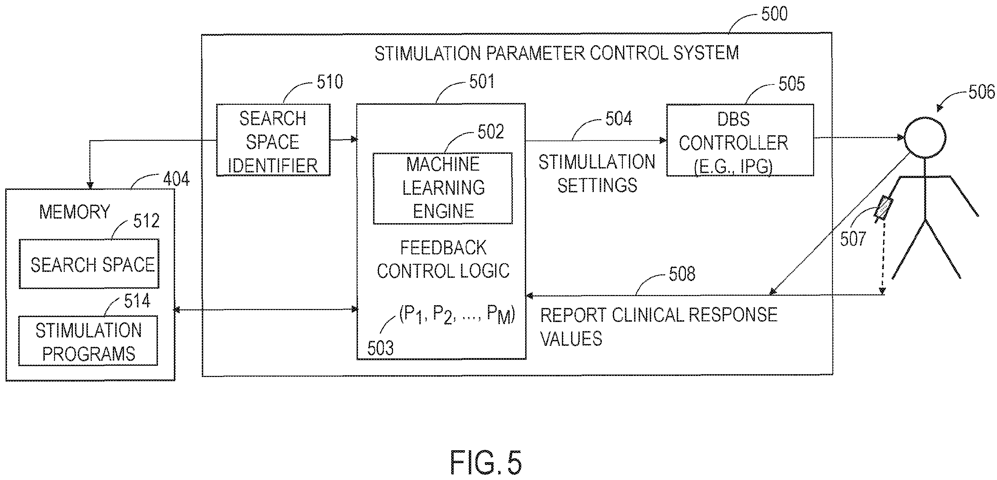

The computing device 400, also referred to as a programming device, can be a computer, tablet, mobile device, or any other suitable device for processing information. The computing device 400 can be local to the user or can include components that are non-local to the computer including one or both of the processor 402 or memory 404 (or portions thereof). For example, the user may operate a terminal that is connected to a non-local processor or memory. In some examples, the computing device 400 can include a watch, wristband, smartphone, or the like. Such computing devices can wirelessly communicate with the other components of the electrical stimulation system, such as the CP 129, RC 128, ETM 130, or IPG 127 illustrated in FIG. 1. The computing device 400 may be used for gathering patient information, such as general activity level or present queries or tests to the patient to identify or score pain, depression, stimulation effects or side effects, cognitive ability, or the like. In some examples, the computing device 400 may prompt the patient to take a periodic test (for example, every day) for cognitive ability to monitor, for example, Alzheimer's disease. In some examples, the computing device 400 may detect, or otherwise receive as input, patient clinical responses to electrostimulation such as DBS, and determine or update stimulation parameters using a closed-loop algorithm based on the patient clinical responses, as described below with reference to FIG. 5. Examples of the patient clinical responses may include physiological signals (e.g., heart rate) or motor parameters (e.g., tremor, rigidity, bradykinesia). The computing device 400 may communicate with the CP 129, RC 128, ETM 130, or IPG 127 and direct the changes to the stimulation parameters to one or more of those devices. In some examples, the computing device 400 can be a wearable device used by the patient only during programming sessions. Alternatively, the computing device 400 can be worn all the time and continually or periodically adjust the stimulation parameters. In an example, the closed-loop algorithm for determining or updating stimulation parameters can be implemented in a mobile device, such as a smartphone, that is connected to the IPG or an evaluating device (e.g. a wristband or watch). These devices can also record and send information to the clinician.

The processor 402 can include one or more processors that may be local to the user or non-local to the user or other components of the computing device 400. In an example, the processor 402 may execute instructions (e.g., stored in the memory 404) to determine a search space of electrode configurations and parameter values, and identify or update one or more stimulation settings that are selectable for use in electrostimulation therapies such as DBS. In this document, the search space refers to a collection of available electrodes, possible electrode configurations, and possible values or value ranges of one or more stimulation parameters that may be applied to selected electrodes to deliver electrostimulation. The search space can be specific to a particular lead or a type of lead (such as lead 218 or lead 219) with respect to a specific neural target. As a result, for different leads or types of lead and/or for different neural targets, the processor 402 may determine respective different search spaces. A stimulation setting includes an electrode configuration and values for one or more stimulation parameters. The electrode configuration may include information about electrodes (ring electrodes and/or segmented electrodes) selected to be active for delivering stimulation (ON) or inactive (OFF), polarity of the selected electrodes, electrode locations (e.g., longitudinal positions of ring electrodes along the length of a non-directional lead, or longitudinal positions and angular positions of segmented electrodes on a circumference at a longitudinal position of a directional lead), stimulation modes such as monopolar pacing or bipolar pacing, etc. The stimulation parameters may include, for example, current amplitude values, current fractionalization across electrodes, stimulation frequency, stimulation pulse width, etc.

The processor 402 may identify or modify a stimulation setting from the search space through an optimization process until a search criterion is satisfied, such as until an optimal, desired, or acceptable patient clinical response is achieved. In an example, the processor 402 may identify a first set of base stimulation settings and a different second set of base stimulation settings. Each base stimulation setting of the first and second sets comprises an electrode configuration and stimulation parameter values or value ranges selected from the search space. For a base stimulation setting of the first set, electrostimulation programmed with said base setting can be delivered to the patient, clinical effects (including therapeutic effects and/or side effects, or motor symptoms such as bradykinesia, tremor, or rigidity) may be detected, and a clinical response be evaluated based on the detected clinical effects. Because actual electrostimulation is administered, the base stimulation settings of the first set are referred to as tested base stimulation settings, and the clinical responses are referred to as tested clinical responses. In contrast, for a base stimulation setting of the second set, no electrostimuation is delivered to the patient; instead, clinical effects may be predicted using a computational model based at least on the clinical effects detected from the electrostimulation testing based on the first set of base stimulation settings, and a clinical response may be estimated using the predicted clinical effects. Because no electrostimulation is delivered, the base stimulation settings of the second set are referred to as predicted or estimated base stimulation settings, and the clinical responses are referred to as predicted or estimated clinical responses. Examples of identifying the first and second sets of stimulation settings and providing electrostimulation therapy in accordance with such an identified stimulation setting are discussed below with reference to FIG. 5.

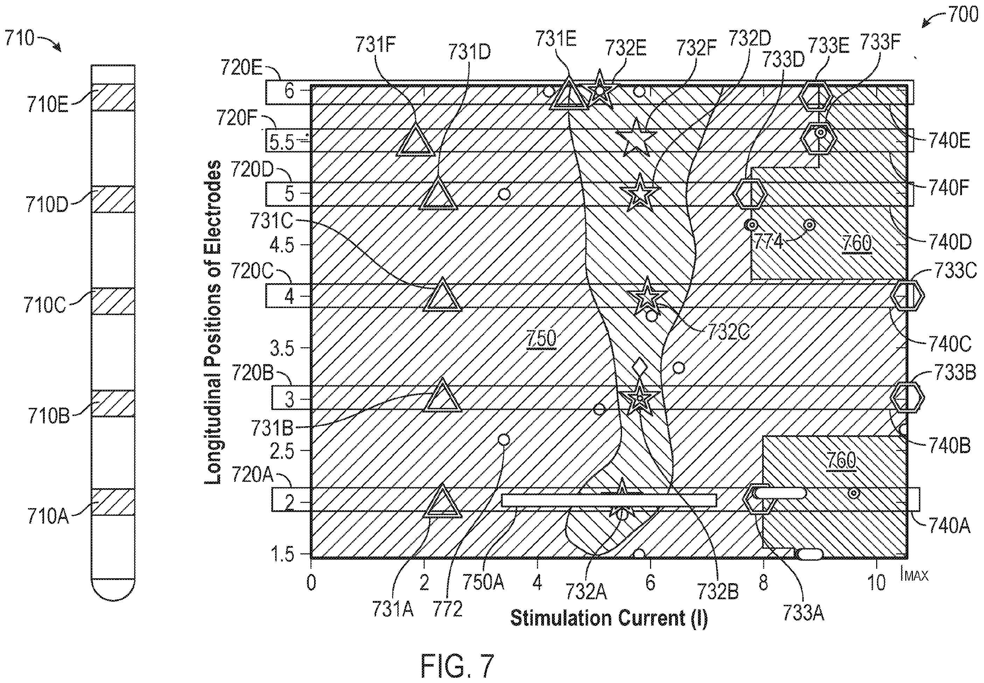

The processor 402 may determine one or more characteristic stimulation amplitudes for an electrode configuration in the search space, such as for an electrode configuration of a base stimulation setting of a first set, or an electrode configuration of a base stimulation setting of a second set. The characteristic stimulation amplitudes each correspond to respective clinical response indicators or clinical effects satisfying respective conditions. The characteristic stimulation amplitudes can be determined based on the clinical response indicators of the first and second sets of base stimulation settings. In some examples, characteristic stimulation amplitudes can be determined for each of a range of electrodes on a lead, such as ring electrodes on the lead 218, or segmented electrodes on the lead 219. The processor 402 can further generate a formatted report including the characteristic stimulation amplitudes for one or more electrodes or electrode configurations, optionally along with the clinical response data of the first and second sets of base stimulation settings. The formatted report can be presented to a system user (e.g., a physician) to assist programming of electrostimulation such as DBS. Examples of generating characteristic stimulation amplitudes and presenting a formatted report are discussed below with reference to FIGS. 7-8.

In various examples, portions of the functions of the processor 402 may be implemented as a part of a microprocessor circuit. The microprocessor circuit can be a dedicated processor such as a digital signal processor, application specific integrated circuit (ASIC), microprocessor, or other type of processor for processing information. Alternatively, the microprocessor circuit can be a processor that can receive and execute a set of instructions of performing the functions, methods, or techniques described herein.

The memory 404 can store instructions executable by the processor 402 to perform varies functions including, for example, determining a reduced or restricted electrode configuration and parameter search space (also referred to as a "restricted search space"), creating or modifying one or more stimulation settings within the restricted search space, etc. The memory 404 may store the search space, the first set of base stimulation settings (i.e., the "tested" stimulation settings) and the second set of base stimulation settings (i.e., the "predicted" or "estimated" stimulation settings), clinical effects (e.g., therapeutic effects and/or side effects) and clinical responses corresponding to the base stimulation settings of the first and second sets, and the characteristic stimulation amplitudes for one or more electrodes or electrode configurations on the lead.

The memory 404 can be a computer-readable storage media that includes, for example, nonvolatile, non-transitory, removable, and non-removable media implemented in any method or technology for storage of information, such as computer readable instructions, data structures, program modules, or other data. Examples of computer-readable storage media include RAM, ROM, EEPROM, flash memory, or other memory technology, CD-ROM, digital versatile disks ("DVD") or other optical storage, magnetic cassettes, magnetic tape, magnetic disk storage or other magnetic storage devices, or any other medium which can be used to store the desired information, and which can be accessed by a computing device.

Communication methods provide another type of computer readable media; namely communication media. Communication media typically embodies computer-readable instructions, data structures, program modules, or other data in a modulated data signal such as a carrier wave, data signal, or other transport mechanism and include any information delivery media. The terms "modulated data signal," and "carrier-wave signal" includes a signal that has one or more of its characteristics set or changed in such a manner as to encode information, instructions, data, and the like, in the signal. By way of example, communication media includes wired media such as twisted pair, coaxial cable, fiber optics, wave guides, and other wired media and wireless media such as acoustic, RF, infrared, Bluetooth.TM., near field communication, and other wireless media.

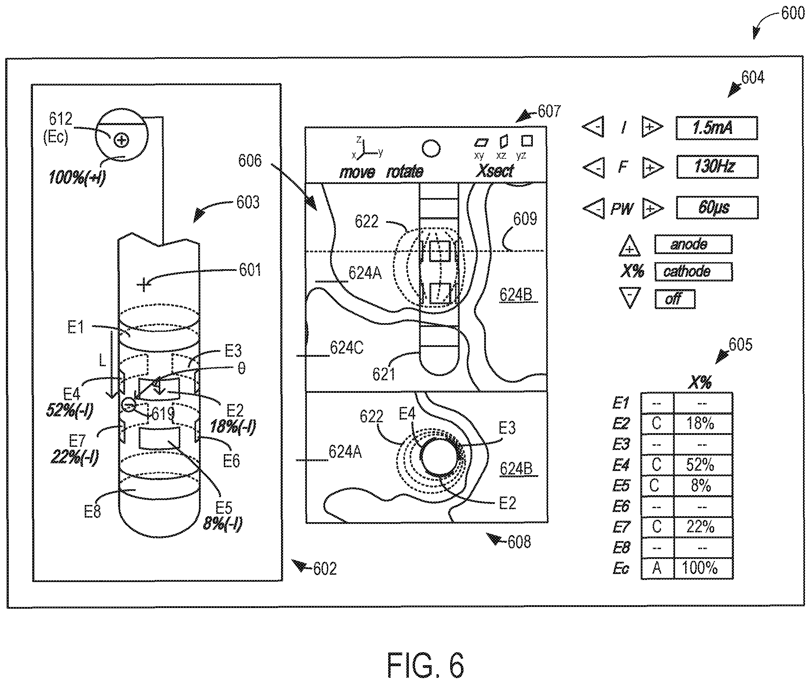

The display 406 can be any suitable display or presentation device, such as a monitor, screen, display, or the like, and can include a printer. The display 406 can be a part of a user interface configured to display information about stimulation settings (e.g., electrode configurations and stimulation parameter values and value ranges) and user control elements for programming a stimulation setting into an IPG such as that shown in FIG. 6. In an example, the display 406 can be configured to display a graphical representation of characteristic stimulation amplitudes for one or more electrode configurations, along with the clinical response data of the first and second sets of base stimulation settings, such as those illustrated in FIGS. 7-8. In some examples, the user interface may include a speaker that provides audio information about stimulation settings, and receive audio input from the user, such as a clinician or the patient.

The input device 408 can be, for example, a keyboard, mouse, touch screen, track ball, joystick, voice recognition system, or any combination thereof, or the like. Another input device 408 can be a camera from which the clinician can observe the patient. Yet another input device 408 is a microphone where the patient or clinician can provide responses or queries.

The electrical stimulation system 412 can include, for example, any of the components illustrated in FIG. 1. The electrical stimulation system 412 may communicate with the computing device 400 through a wired or wireless connection or, alternatively or additionally, a user can provide information between the electrical stimulation system 412 and the computing device 400 using a computer-readable medium or by some other mechanism. As mentioned, in some examples, the computing device 400 may include part of the electrical stimulation system, such as, for example, the IPG 127, RC 128, CP 129, or ETM 130 or any combination thereof.

FIG. 5 illustrates, by way of example and not limitation, a stimulation parameter control system 500 and a part of the environment in which it may operate. The stimulation parameter control system 500, which may be implemented as a part of the processor 402, may include a feedback control logic 501, a DBS controller 505, and a search space identifier 510. The feedback control logic 501 may be implemented in, for example, the CP 129 or the RC 128. The feedback control logic 501 can determine or modify one or more stimulation settings 504 for a stimulation lead (such as leads 218 or 219) at target stimulation region, such as a region in a brain hemisphere. A stimulation setting includes an electrode configuration and values for one or more stimulation parameters (P₁, P₂, ..., Pₘ) 503. The electrode configuration includes information about electrodes (ring electrodes and/or segmented electrodes) selected to be active for delivering stimulation (ON) or inactive (OFF), polarity of the selected electrodes, electrode locations (also referred to as contact locations, which may include longitudinal positions of ring electrodes along the length of a lead, or angular positions of segmented electrodes about a circumference of a cross-section of the lead at a longitudinal position), and stimulation modes (e.g., monopolar pacing or bipolar pacing), etc. The stimulation parameters may include, for example, current amplitude values, current fractionalization across electrodes, stimulation frequency, stimulation pulse width, etc. In some examples, the feedback control logic 501 may modify the stimulation setting 504 such as by changing a stimulation parameter value, or modifying an electrode configuration.

The stimulation setting 504 may be provided to the DBS controller 505 to configure the IPG 127 or ETM 130 to deliver DBS therapy to the patient 506 in accordance with the stimulation setting or the modified stimulation setting. The stimulation may produce certain therapeutic effects and/or side effects on the patient 506. Such therapeutic effectiveness and side effects, also referred to as clinical responses or clinical metrics, may be provided to the feedback control logic 501. In an example, the clinical responses may be based on patient or clinician observations. For example, motor symptoms such as bradykinesia (slowness of movement), rigidity, tremor, among other symptoms or side effects, can be scored by the patient or by the clinician upon overserving or questioning the patient. In some examples, the clinical responses can be objective in nature, such as measurements automatically or semi-automatically taken by a sensor 507. In an example, the sensor 507 may be included in a wearable device associated with patient 506, such as a smart watch. For example, a Parkinson's patient may be fitted with a wearable sensor that measures tremors, such as by measuring the frequency and amplitude of such tremors. U.S. Patent Application Serial No. 17/137,110, filed December 29, 2020, which is incorporated herein by reference in its entirety, discusses determining which symptoms and/or side effects are most sensible to score for a given patient when the stimulation parameters are optimized.

The clinical responses, either reported by the patient or measured by a sensor, may be converted to clinical response values 508, also referred to as clinical response scores. In an example, the clinical response values 508 may be computed based on the intensity, frequency, or duration of one or more of tremor, rigidity, or bradykinesia responses. Based upon the received clinical response values 508, the feedback control logic 501 can adjust electrode configurations or values of one or more stimulation parameters 503. The feedback control logic 501 can send the adjusted (new or revised) stimulation setting 504, such as the electrode configuration or the adjusted stimulation parameter values, to further configure the DBS controller 505 to change the stimulation parameters of the leads implanted in patient 506 to the adjusted values.

The feedback-control loop as illustrated in FIG. 5 can continue until an optimal, desired, or acceptable outcome is reached, such as maximizing therapeutic effectiveness while minimizing unwanted side effects, or until a specific stop condition is reached such as number of iterations, time spent in programming session, or the like. An outcome may be considered optimal, desired, or acceptable if it meets certain threshold values or tests (e.g., improved clinical response for the patient, faster programming of the device, increased battery life, and/or control multiple independent current sources and directional lead). Such an iterative process of looking for a stimulation setting (e.g., an electrode configuration and stimulation parameter values for the electrode) is referred to as a stimulation setting optimization process. The outcome being reached is referred to as an optimization criterion, and the resultant stimulation setting is referred to as an optimal base stimulation setting (BSS). By way of example and not limitation, the optimization criterion may include possible optimal clinical outcome within the parameters chosen; time spent, iterations taken, or power usage to explore the search space until a desired clinical outcome is reached (assuming multiple outcomes with the same or comparable clinical response); among others.

In an example, the optimization criterion includes the clinical response values 508 exceeding a threshold value or falling into a specified value range, indicating a satisfactory therapeutic outcome has reached. Depending on how the clinical response values are computed, one or more optimal base stimulation settings may be determined. For example, the clinical response values may be computed using a single response effect (e.g., one of bradykinesia, tremor, or rigidity). Accordingly, three optimal base stimulation settings may be generated: a first optimal base stimulation setting (BSS₁) corresponding to a bradykinesia score exceeding a threshold, a second optimal base stimulation setting (BSS₂) corresponding to a tremor score exceeding a threshold, and a third optimal base stimulation setting (BSS₃) corresponding to a rigidity score exceeding a threshold. In another example, the clinical response values can be a composite score computed as a weighted combination of multiple clinical effects, such as a%* bradykinesia + b%* tremor + c%* rigidity. Accordingly, a fourth optimal base stimulation setting (BSS₄) can be generated, corresponding to the composite clinical response score exceeding a threshold. In some examples, the stimulation setting optimization can be performed in an in-clinic programming session such during implantation or revision of a DBS system or device follow-up, where a clinician may use a graphical user interface (GUI) interacting with the feedback control logic 501 to generate one or more optimal base stimulation settings, as discussed below with reference to FIG. 6.

The optimal base stimulation settings (e.g., BSS₁ through BSS₄), may be stored in the memory 404. In an example, a stimulation setting, along with the corresponding unique clinical response indicator (e.g., weighted combination of clinical effects with unique weight factors) form a stimulation program 514, which can also be stored in the memory 404. Each stimulation programmed can be associated with, or tagged by, one or more unique clinical response indicators.

In some examples, the clinical response values 508 may be weighted according to the time at which the test took place. It has been found that, for at least some patients or at least some testing procedures, patient fatigue occurs over time which may degrade or otherwise change the clinical response values (whether obtained from the patient, clinician, or a sensor). For example, more recent clinical response values 508 may be weighted higher than earlier clinical response values as the more recent clinical response values are more predictive of the clinical response to the adjusted stimulation parameters because these later tests have a similar amount of patient fatigue. By way of example and not limitation, the weights can be in a range from 1.05 to 1.5.

In various examples, the stimulation parameter control system 500 may be executed on its own and is not connected to a controller. In such instances it may be used to merely determine and suggest programming parameters, visualize a parameter space, test potential parameters, etc.

The process of iterative search for a stimulation setting (e.g., an electrode configuration and/or stimulation parameter values) typically involves significant computation and time, especially when electrode configuration involves segmented electrodes in a directional lead (e.g., lead 219). If testing all possible settings in the entire parameter space (including electrode configurations and combinations of stimulation parameter values) is done as comprehensively as possible, stimulation would need to be provided to the patient for each possible setting, which may end up with a burdensome and time-consuming programming session. Because practically a programming session may only last a few hours, only a fraction of possible electrode configuration and stimulation parameter combinations can reasonably be tested and evaluated. To reduce the time taken and to improve the efficiency of stimulation setting optimization process, a reduced or restricted electrode configuration and parameter search space can be used. By applying limitations or constraints to the electrode configurations and parameter values, the restricted search space can include a subset of electrodes (e.g., a subset of ring electrodes and/or a subset of segmented electrodes on a lead) that are selected as active electrodes for delivering stimulation, and values or value ranges for one or more stimulation parameters (e.g., a range of current amplitude ranges for an active electrode). Stimulation setting optimization, when performed within such a search space, can be more efficient and cost-effective than searching through the entire parameter space for one or more optimal base stimulation settings such as BSS₁ - BSS₄ as discussed above.

The search space identifier 510 can automatically determine a search space 512 for a stimulation lead (e.g., leads 218 or 219) at a neural target, such as a region in a brain hemisphere, by imposing certain limitations or constraints on the electrode configurations and/or parameter values or value ranges. In an example, the search space 512 can be determined based on spatial information of the lead, such as lead positions with respect to neural targets, which can be obtained from imaging data of the lead and patient anatomy. Additionally or alternatively, the search space 512 can be determined based on physiological information such as physiological signals sensed by the electrodes at their respective tissue contact locations. The physiological information may include patient clinical responses to stimulation. In some examples, prior knowledge about patient medical condition, health status, DBS treatment history may also be utilized to determine the search space 512. In an example, the search space identifier 510 may exclude those electrodes on the lead that are out of a region of interest, such that the search space includes only those electrodes within the target of interest. One or more stimulation parameters may be restricted to take certain values or within value ranges. For example, the restricted search space may include certain electrode positions and value ranges for stimulation current amplitude, frequency, or pulse width. The feedback control logic 501 can determine one or more optimal base stimulation settings (e.g., BSS₁ - BSS₄) by searching through the identified search space 512. The identified search space 512 can be stored in the memory 404. Commonly assigned U.S. Patent Application Serial No. 63/186,596, filed May 10, 2021, discusses determining a search space and adjusting electrode configuration and/or parameter values within the search space. Commonly assigned U.S. Patent Application Serial No. 63/186,590, filed May 10, 2021, discusses reducing neurostimulation electrode configuration and parameter search space based on electrode position information relative to an anatomical region of interest or physiological signals sensed by the plurality of electrodes, and determining a target stimulation setting based on patient clinical responses.

The feedback control logic 501 may include a machine learning engine 502 that can facilitate the stimulation parameter control system 500 (or a user of the system) to explore the search space in order to choose values for programming the DBS controller 505. The machine learning engine 502 can employ supervised or unsupervised learning algorithms to train a prediction model, and use the trained prediction model to predict patient clinical responses to an untested stimulation setting (e.g., untested stimulation parameter values or untested electrode configurations), or to estimate or predict stimulation parameters values or electrode configurations that, when provided to the DBS controller 505 to deliver stimulation accordingly to the patient 506, would produce desired or improved clinical responses. Examples of the learning algorithms include, for example, Naive Bayes classifiers, support vector machines (SVMs), ensemble classifiers, neural networks, Kalman filters, regression analyzers, etc. The machine learning engine 502 can build and train a prediction model using training data, such as stimulation parameter values and corresponding patient clinical responses. The training date can be acquired from a training session such as performed in a clinic. Additionally or alternatively, the training data can be obtained from historical data acquired by the stimulation parameter control system 500. With its learning and prediction capability, the machine learning engine 502 can aid a user (e.g., a clinician) in exploring the stimulation parameter space more effectively and more efficiently to produce results that are optimal, desired, or acceptable.

In some examples, the machine learning engine 502 can utilize imaging data to inform the choice of the next set of values. This will be particularly useful, when the algorithm finds itself in a region of parameter space for which the clinical responses are not substantially affected by the changes in the stimulation parameters, and the choice of next step is not apparent from the patient response alone. Imaging data that provides information about the location of the lead in the patient's brain along with priors informing the algorithm of which directions may be better choices for the next step could lead to faster convergence.

In some examples, the machine learning engine 502 can determine expected outcomes for parameter values that have not yet been tested based upon what the machine learning engine 502 has "learned" thus far, and provide a recommendation for a next set of values to test. Here, testing refers to the iterative testing required to find an optimal stimulation setting for configuring the DBS controller 505. The recommendation for a next set of values to test is based upon which of the determined expected outcomes meet a set of designated (determined, selected, preselected, etc.) criteria (e.g., rules, heuristics, factors, and the like). For example, rules considered may include such factors as: the next set of values cannot be one of the last 10 settings tested or cannot be too close to previously tested setting. Accordingly, the feedback control logic 501 with its machine learning engine 502 is used to systematically explore the stimulation parameter space based upon what it has learned thus far and (optionally) different rules and/or heuristics that contribute to achieving optimal outcomes more efficiently.

The process for determining expected outcomes for parameter values that have not yet been tested may involve use of other data for machine learning. For example, data from other programming sessions for the same patient as well as from other patients may be used to train the machine learning engine 502. In some examples, no prior data may be used. In this case, the machine learning engine 502 may use data learned from this patient only in one particular setting. In other examples, data from the same patient but from previous sessions may be used. In some examples all patient data from all sessions may be used. In some examples all patient data utilizing lead location information (knowledge of lead location in space relative to anatomy) may be used. Different other combinations are also possible.

In order to use this data for machine learning purposes, the data may first be cleansed, optionally transformed, and then modeled. In some examples, new variables are derived, such as for use with directional leads, including central point of stimulation, maximum radius, spread of stimulation field, or the like. Data cleansing and transformation techniques such as missing data imputation and dimension reduction may be employed to prepare the data for modeling.

The machine learning engine 502 may determine how best a predicted outcome meets the optimal outcome metrics. Various optimization techniques may be used, examples of which may include but are not limited to: optimization algorithms and estimation procedures used to fit the model to the data (e.g., gradient descent, Kalman filter, Markov chain, Monte Carlo, and the like); optimization algorithms reformulated for search (e.g., simulated annealing); spatial interpolation (e.g., kriging, inverse distance weighting, natural neighbor, etc.); supplementary methods that aid the optimization process (e.g., variable selections, regularization, cross validation, etc.); other search algorithms (e.g., golden-section search, binary search, etc.). Using any of these techniques, the machine learning engine 502 can decide whether a particular predicted outcome for a set of stimulation parameter values is the fastest sufficing outcome, the best possible clinical outcome, or the optimal outcome with least battery usage, for example.

The feedback control logic 501 may be used to search and configure different types of stimulation parameters of the various leads potentially causing different clinical effects upon the patient 506. Examples of the stimulation parameters may include electrode configurations (electrode selection, polarities, monopolar or bipolar modes of stimulation), current fractionalization, current amplitude, pulse width, frequency, among others. Given these possible stimulation parameters, the stimulation parameter control system 500 can move about the parameter space in different orders, by different increments, and limited to specific ranges. In some examples, the stimulation parameter control system 500 may allow the user to provide search range limitations to one or more of the stimulation parameters to limit the range for that stimulation parameter over which the system will search for parameters. For example, the user may restrict which electrodes can be used for stimulation or may restrict the amplitude or pulse width to a certain range or with a selected maximum or minimum. As one illustration, based on the site of implantation, the user may be aware that the distal-most and proximal-most electrodes are unlikely to produce suitable stimulation and the user limits the range of electrodes to exclude these two electrodes.

For a lead with segmented electrodes, the number of possibilities for parameter selection can be very large when combinations of electrodes and different amplitudes on each electrode are possible. In some examples using a lead with segmented electrodes, the selection of electrodes used for stimulation may be limited to fully directional selections (i.e., selection of only a single segmented electrode) and fully concentric selections (i.e., all electrodes in a single set of segmented electrodes are active with the same amplitude). In other examples, the initial movement through parameter space may be limited to fully directional and fully concentric selections. After a set of stimulation parameters is identified using these limits, variation in the selection of electrodes may be opened up to other possibilities near the selection in the identified set of stimulation parameters to further optimize the stimulation parameters.

In some examples, the number of stimulation parameters that are varied and the range of those variations may be limited. For example, some stimulation parameters (e.g., electrode selection, amplitude, and pulse width) may have larger effects when varied than other stimulation parameters (e.g., pulse shape or pulse duration). The movement through stimulation parameter space may be limited to those stimulation parameters which exhibit larger effects. In some examples, as the stimulation parameter control system 500 proceeds through testing of sets of stimulation parameters, the system may observe which stimulation parameters provide larger effects when varied and focus on exploring variation in those stimulation parameters.

In some examples, the stimulation parameter control system 500 can include a user interface for visualizing exploration of the stimulation parameter space as the system determines new and better parameter values to test until a solution is determined that fits within certain designated thresholds or a stop condition is reached. In some examples of the stimulation parameter control system 500, the user interface is part of the feedback control logic 501. In other examples, the user interface may be part of another computing system that is part of the stimulation parameter control system 500 or may be remote and communicatively connected to the stimulation parameter control system 500. The user interface may present to a user (such as a clinician, physician, programmer, etc.) a visualization of the predicted expected outcomes for (some of) the stimulation parameter values not yet tested and a recommendation for the next set of stimulation parameter values to test.

In some examples where a deep brain stimulator is configured via the DBS controller 505 with at least one set of stimulation parameter values forwarded by the feedback control logic 501, the clinician may monitor the patient throughout the process and record clinical observables in addition to the patient 506 being able to report side effects. When a side effect is observed, the various search algorithms will take that fact into account when selecting/suggesting a next set of values to test. In some examples, for example, those that select contacts via monopolar review, other parameters may be changed until they cause a side effect, which case is noted as a boundary. For example, in monopolar review where amplitude is another stimulation parameter being varied, the amplitude may be increased progressively until a side-effect is observed.

In some examples, more than one clinical metric (e.g., tremor, rigidity, bradykinesia, etc.) may be important observables. Different examples of the stimulation parameter control system 500 may handle these metrics differently. For example, some examples might identify an ideal location for each metric and choose one ideal location between them, set in the patient's remote controller so the patient can choose as needed, or chose a best combined outcome. As another example, some examples may search multiple outcomes at the same time and use the best combined score as the best outcome or find a best location for each metric individually. As yet another example, some examples may use a sequential process for selecting stimulation parameter values for multiple outcomes. For example, a system may search parameter space for a first outcome (e.g., bradykinesia) and, upon finding a suitable end condition, then search parameter space for a second outcome (e.g., rigidity). While searching parameter space for the first outcome, clinical response values for both the first and second outcomes can be obtained. Thus, when the system switches to the second outcome there are already a number of clinical response values for that outcome which will likely reduce the length of the search.

In some examples, two stimulation leads may be implanted to produce stimulation effects on two sides of the body (e.g., the right and left sides of the body). The same procedure described herein can be used to either jointly determine the stimulation parameters for the two leads by exploring the joint parameter space or individually determine stimulation parameters for the two leads by exploring the parameter space for each lead individually. In some examples, the user may determine for each side of the body which clinical response is dominant or most responsive. This may be done, for example, by having the patient perform a single task which captures multiple responses (e.g. connecting dots on the screen to monitor tremor and bradykinesia of the movement) or a small series of tasks. This enables the system to determine which clinical response to use to identify the stimulation parameters for that side of the body.

As noted, the feedback may be provided directly by the patient 506, entered by an observer such as a clinician (not shown), or may be provided by means of a sensor 507 associated with and in physical, auditory, or visual contact with the patient 506. In an example, the sensor 507 may be included in a wearable device associated with patient 506, such as a smart watch. In an example where the feedback can be monitored automatically or semi-automatically, such as with use of sensor 507, it may not be necessary for a clinician or other observer to be present to operate the stimulation parameter control system 500. Accordingly, in such examples a user interface may not be present in system 500.

In some examples, the stimulation parameter control system 500 may determine one or more optimal base stimulation settings using predicted clinical responses for untested stimulation parameter values or untested electrode configurations without actually delivering stimulation. Such base stimulation settings are referred to as estimated or predicted base stimulation settings, to distinguish from the tested base stimulation settings (e.g., BSS₁ - BSS₄) that are based on the tested clinical response (either reported by the patient or measured by a sensor) to actually delivered stimulation. For examples, based on the "tested" base stimulation settings BSS₁ - BSS₄, the stimulation parameter control system 500 may estimate an optimal base stimulation setting associated with a composite clinical response defined as x%* bradykinesia + y%* tremor + z%* rigidity, or simply denoted by the weight factors (x%, y%, z%). By way of example and not limitation, the stimulation parameter control system 500 may generate a fifth optimal base stimulation setting (BSS₅) corresponding to a composite clinical response using bradykinesia and tremor only, each weighted 50%; a sixth optimal base stimulation setting (BSS₆) corresponding to a composite clinical response using tremor and rigidity only, each weighted 50%; a seventh optimal base stimulation setting (BSS₇) corresponding to a composite clinical response using bradykinesia and rigidity only, each weighted 50%; or an eighth optimal base stimulation setting (BSS₈) corresponding to a composite clinical response using bradykinesia, tremor, and rigidity weighted 40%, 40%, and 20%, respectively. Similar to the tested base stimulation settings BSS₁ - BSS₄, the estimated base stimulation settings BSS₅ - BSS₈, associated with their respective clinical response indicators (e.g., weight factors for clinical effects), can be stored in the memory 404 as respective stimulation programs 514. In an example, the stimulation programs 514 may be stored in a lookup table, where each tested or estimated base stimulation setting (e.g., BSS₁ through BSS₈) may be tagged by respective clinical response indicators or weight factors for clinical effects. In an example, the memory 404 can be a part of memory circuitry internal to the IPG (e.g., IPG 127 or IPG 210). The RC 128 or the CP 129 can request access to the memory 404 to retrieve therefrom one or more stored stimulation programs 514 or the search space 512.

FIG. 6 illustrates, by way of example and not limitation, a graphical user interface (GUI) 600 operable on an external device such as the clinician program (CP) 129, which allows a user (e.g., a clinician) to program a stimulation setting into the IPG (e.g., IPG 127 or IPG 210). The GUI 600 can be rendered on a display of the CP 129, and provide a clinician with a visual indication of how a stimulation setting (e.g., electrode configuration and/or values for a plurality of stimulation parameters) may interact with target tissue (e.g., brain tissue in the context of DBS) in which the electrodes are implanted. The GUI 600 may be used during surgical implantation of the leads 218 or 219 and the IPG 210, but may also be used after implantation to assist in creating, modifying, or selecting a therapeutically useful stimulation program for the patient.

The GUI 600 can be controlled by a cursor 601 that the user can move using a tracking device such as a mouse connected to the CP 129. The GUI 600 may include a stimulation waveform interface 604 that allows a user to select, and adjust a value of, a stimulation waveform parameter, such as a stimulation amplitude (e.g., a current I), a frequency (F), or a pulse width (PW) of stimulation pulses. In some examples, the waveform interface 604 can be significantly more complicated, particularly if the IPG 210 supports the provision of stimulation that is more complicated than a repeating sequence of pulses. In some examples, the waveform interface 604 may allow a user to select biphasic or monophasic pulses, and to select whether passive charge recovery will be used (not shown).

The GUI 600 may include an electrode configuration interface 605 which allows the user to select and modify a particular electrode configuration, such as specifying which electrodes are active electrodes (ON) to provide stimulation, and which electrodes are inactive electrodes (OFF) to refrain from providing stimulation. For an active electrode, the user may also use the electrode configuration interface 605 to specific polarity and relative magnitude for that active electrode. As illustrated in FIG. 6, the user may use the electrode configuration interface 605 to designate an electrode as an anode (A), a cathode (C), or an inactive electrode (OFF), and to specific an amount of the total anodic or cathodic current +I or -I (specified in the waveform interface 604) that a selected active electrode will receive in a form of fraction or percentage (X%) of the total current provided. Such a split of current among multiple electrodes is referred to as current fractionalization. In an example as illustrated in FIG. 6, the case electrode 612 (Ec) is specified as the only anode that receives 100% of anodic current +I. The corresponding cathodic current -I is split among a set of selected active cathode electrodes including E2 (18% of -I), E4 (52% of -I), E5 (8% of -I), and E7 (22% of -I). One or more electrodes can be chosen to act as anodes or cathodes at a given time, allowing the electric field in the tissue to be shaped. Once the stimulation waveform interface 604 and electrode configuration interface 605 are determined, they can be sent to the IPG 210, which can produce stimulation pulses accordingly, and deliver the stimulation pulses to the patient.

The GUI 600 can include a leads interface 602 showing an image 603 of the lead being used for the patient. By way of example and not limitation, the lead shown in the image 603 includes two ring electrodes E1 and E8, a first group of segmented electrodes E2-E4 about a circumference of a first longitudinal position of the lead, and a second group of segmented electrodes E5-E7 about a circumference of a second longitudinal position of the lead. In this example, segmented electrodes E2, E4, E5, and E7 are selected as cathodes to receive fractionalized current. Such current fractionalization sets a particular position for a cathode pole 619 in a three-dimensional space. The position of this cathode pole 619 can be quantified at a particular longitudinal position L along the lead (e.g., relative to a point on the lead such as the longitudinal position of electrode E1). If a direction lead such as lead 219 is uses, the position of the cathode pole 619 may further be quantified at a particular rotational angle θ (e.g., relative to a particular angle on the lead such as relative to the center of electrode E2). The position (L, θ) of the cathode pole 619, as shown in the leads interface 602, may be virtual; that is, the position may not necessarily be at a physical position of any electrode on the lead, but a point in the three-dimensional space of the leads interface 602. In some examples, the interface 602 can include a selection to access a library of images 603 of the types of leads (e.g., 218 or 219) that may be implanted in different patients, which may be stored with the CP 129. The cursor 601 can be used to select an electrode such as any of E1-E8, or the case electrode Ec, or a pole such as cathode pole 619.

The CP 129 can include and execute an electrode configuration algorithm to determine a position of the cathode pole 619 in the three-dimensional space from a given electrode configuration, or determine an electrode configuration from a given position of the cathode pole 619. For example, the user can place the position of the cathode pole 619 using the cursor 601. The electrode configuration algorithm can then be used to compute an electrode configuration (e.g., current fractionalization across a plurality of selected active electrodes) that best places the cathode pole 619 in this position. The electrode configuration algorithm may thus calculate that electrode E4 should receive the largest share of cathodic current (52%*-I), while E2, E7, and E6 which are farther away from the cathode pole 619 receive lesser percentages, as shown in the stimulation parameters interface 604. By involving more than one electrode, cathode pole 619 is formed as a virtual pole not as the position of any of the physical electrodes. Again, the electrode configuration algorithm can also operate in reverse: from a given electrode configuration, the position of the cathode pole 619 can be determined. The electrode configuration algorithm is described further in U.S. Patent Application Publication 2019/0175915.

The GUI 600 can further include a visualization interface 606 that allows a user to view a stimulation field image 622 formed on a lead given the selected stimulation parameters and electrode configuration. The stimulation field image 622 is formed by field modelling in the CP 129. The visualization interface 606 may include tissue imaging information, such as imaging information of different brain tissue structures 624A, 624B and 624C in the context of DBS. Such tissue imaging information may come from a Magnetic Resonance Image (MRI) or Computed Tomography (CT) image of the patient, may come from a generic library of images, and may include user defined regions. The GUI 600 can overlay the lead image 621 and the stimulation field image 622 with the tissue imaging information in the visualization interface 606 so that the position of the stimulation field image 622 relative to the various tissue structures 624A-624C can be visualized. The various images shown in the visualization interface 606 (i.e., the lead image 621, the stimulation field image 622, and the tissue structures 624A-624C) can be three-dimensional in nature, and hence may be rendered to allow such three-dimensionality to be better appreciated by the user, such as by shading or coloring the images, etc. A view adjustment interface 607 may allow the user to use the cursor 601 to move or rotate the images. As illustrated in FIG. 6, a cross-section interface 608 allows the various images to be seen in a particular two-dimensional cross section, such as a cross section 609 taken perpendicularly to the lead image 621 and through the segmented electrodes E2, E3, and E4.

The GUI 600 can be particularly useful because it allows the electric field as reflected in stimulation field image 622 to be seen relative to surrounding tissue structures 624A-624C. This allows the user to adjust the stimulation parameters to recruit, or avoid recruiting, particular tissue structures. Assume for example that it is desirable for a given patient to stimulate tissue structure 624A, but to not stimulate tissue structures 624B or 624C where the stimulation may cause undesired side effects. The clinician can then use the GUI 600 to adjust stimulation (e.g., to adjust the stimulation parameters or the electrode configuration) to move the stimulation field (e.g., the cathode pole 619) to a proper position (L, θ). In the example shown, and as best seen in the cross-section interface 608, higher cathodic currents are provided at electrodes E4 (52%*-I) and E2 (18%*-I), because these electrodes are generally facing towards tissue structure 624A where stimulation is ideally applied. By contrast, electrode E3 carries no cathodic current because it generally faces towards tissue structure 624B where stimulation is ideally avoided. The result is a stimulation field that is more predominant in tissue structure 624A and less predominant in tissue structure 624B, as shown in the visualization interface 606.

Especially in a DBS application, it is important that correct stimulation parameters be determined for a given patient. Improper stimulation parameters may not yield effective relief of a patient's symptoms, or may cause unwanted side effects. To determine proper stimulation, a clinician may use the GUI 600 to try different combinations of stimulation parameters and electrode configurations. This may occur, during a DBS patient's surgery when the leads are being implanted, or during a post-surgery office visit after the patient has had a chance to heal and after the position of the leads stabilize in the patient. During an in-clinic programming session, a clinician may use the GUI 600 that interacts with the feedback control logic 501 to generate one or more optimal base stimulation settings, as discussed above with reference to FIG. 5.

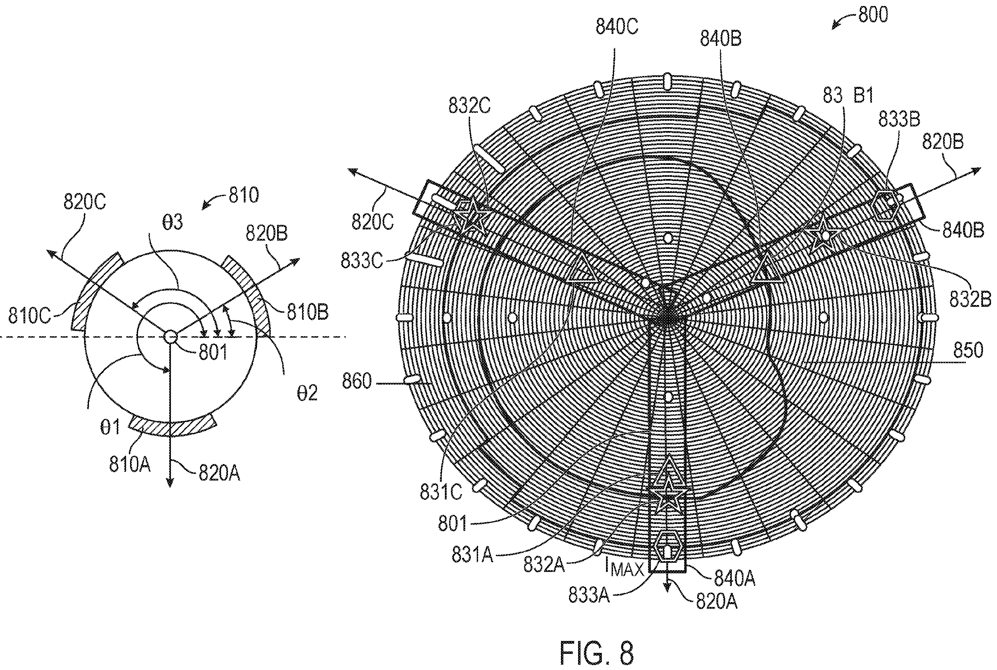

FIGS. 7-8 each illustrate, by way of example and not limitation, respective two-dimensional (2D) monopolar review diagrams of an electrode configuration and parameter search space (the "search space") and a formatted monopolar review of characteristic stimulation amplitudes for one or more electrode configurations in the search space. Specifically, FIG. 7 illustrates a diagram 700 of a search space and a monopolar review of characteristic stimulation amplitudes corresponding to ring mode stimulation. The ring mode stimulation can be delivered using a ring electrode. Alternatively, the ring mode stimulation can be delivered using multiple segmented electrodes around a circumference of the lead, where stimulation current is spread substantially evenly to the segmented electrodes. FIG. 8 illustrates a diagram 800 of a search space and a monopolar review of characteristic stimulation amplitudes corresponding to directional mode stimulation, where separate and non-identical stimulation currents are applied to respective segmented electrodes.

The search space defines a sub-space of electrode configurations and stimulation parameter values that may be used during the stimulation setting optimization to search for one or more optimal base stimulation settings. As discussed above, the search space may be identified, such as by the search space identifier 510, for a lead with known electrodes types and locations on the lead positioned at a target stimulation region such as a region in a brain hemisphere. The search space can be represented by a heatmap that depicts intensities of clinical responses measured or estimated over a range of electrode positions, such as longitudinal locations of the ring electrodes (as shown in FIG. 7) or angular locations of the segmented electrodes (as shown in FIG. 8) relative to the leads geometry and over a range of stimulation parameter values such as current amplitudes (represented by data points on a grayscale or colormap). The intensities of clinical responses may be represented by clinical response scores computed using, for example, a weighted combination of one or more clinical effects (e.g., bradykinesia, tremor, or rigidity). Clinical effects or clinical response scores may be evaluated for a plurality of electrodes at different locations, such as those electrodes involved in a first set of tested base stimulation settings (e.g., BBS₁ - BBS₄ as discussed above with reference to FIG. 5. The clinical effects or clinical response scores may be evaluated in response to stimulation actually delivered with respective stimulation current amplitudes. Such actually tested clinical effects or the clinical response scores can be plotted as data points on the diagrams 700 and 800.

In addition to the actually tested clinical effects (in response to stimulation), clinical effects may be predicted for untested electrode configurations and untested stimulation parameter values, such as a second set of estimated base stimulation settings (e.g., BBS₄ - BBS₈ as discussed above with reference to FIG. 5), without actually delivering electrostimulation. The prediction can be carried out using the machine learning engine 502 of the feedback control logic 501 as discussed above with reference to FIG. 5. Similar to the actually tested clinical effects, the predicted clinical effects or the clinical response scores may be plotted as data points on the diagram 700 or 800.

The clinical effects (including both the tested and the predicted clinical effects as discussed above) may include both positive effects and negative effects. The diagram 700 can include a positive effect region 750 covering substantially data points representing actually tested and predicted positive effects, and a negative effect region 760 covering substantially data points representing actually tested and predicted negative effects. Similarly, the diagram 800 can include a positive effect region 850 covering substantially data points representing actually tested and predicted positive effects, and a negative effect region 860 covering substantially data points representing actually tested and predicted negative effects. The positive effect regions 750 and 850 each define a subset of the search space from which stimulation parameters and electrode configurations may be selected for an electrostimulation therapy without causing undesirable side effects. The negative effect regions 760 and 860 each define a subset of the search space of the stimulation parameters and electrode configurations that need to be avoided for use in an electrostimulation therapy to prevent undesirable side effects.

FIGS. 7-8 each also illustrate characteristic stimulation amplitudes for one or more electrode configurations in the search space. By way of example and not limitation, monopolar configurations are considered in the illustrated examples. The monopolar configuration can include a single ring electrode or a single segmented electrode as cathode, and the device case 212 as anode. In some examples, by spreading cathodic current substantially evenly to a set of segmented electrodes around a circumference of the lead at a given longitudinal position, the segmented electrodes can be jointly configured as cathode, and the device case 212 as anode in a monopolar configuration. The characteristic stimulation amplitudes can be graphically displayed over the search space represented by a heatmap of the intensities of clinical responses measured or estimated over a range of electrode positions. FIG. 7-8 each depict respective monopolar review diagrams (or monopolar review formatted reports) that be displayed to a user (e.g., a physician) and help the user better understand clinical effects of electrostimulations with a wide range of tested and untested electrode configurations and parameter values or value ranges, and guide the user to better program electrostimulation therapy.

The characteristic stimulation amplitudes can be determined for one or more electrode configurations in the search space, such as electrode configurations included in the first set of "tested" base stimulation settings, or electrode configurations included in a second set of "estimated" base stimulation settings. The processor 402 can determine the characteristic stimulation amplitude as current amplitude that, when applied to a specific electrode configuration, would result in clinical response or clinical effects satisfying a specific condition. In some examples, the characteristic stimulation amplitude may be represented by stimulation parameters other than the current amplitude, such as voltage amplitude or stimulation energy applied to a specific electrode configuration.

FIG. 7 illustrates a two-dimensional (2D) monopolar review diagram 700 of at least a portion of the electrode configuration and parameter search space comprising a plurality of ring electrodes 710A-710E at respective different ring mode stimulation positions 720A-720E of a non-directional lead 710 (an example of the non-direction lead 218). The diagram 700 may also be used to illustrate an electrode configuration and parameter search space corresponding to segmented electrodes at a given longitudinal level of a directional lead (e.g., the directional lead 219) where current is spread approximately evenly to the segmented electrodes at that level, such that the segmented electrodes effectually deliver ring mode stimulation. The longitudinal positions of electrodes delivering ring mode stimulation (e.g., ring electrodes, or segmented electrodes jointly to deliver ring mode stimulation) are shown on the y-axis of the diagram 700. The x-axis represents a stimulation amplitude (e.g., current amplitude in milliamps (mA) up to an amplitude limit (Iₘₐₓ) set by a user) of a ring mode stimulation involving a ring electrode or a set of segmented electrodes at a given longitudinal position on the lead. For each ring mode stimulation position along the lead, multiple characteristic stimulation amplitudes, such as determined by the processor 402, can be graphically represented by markers linearly arranged along a direction with respect to the corresponding longitudinal position of the electrode(s) delivering the ring mode stimulation. In the illustrated example, for ring electrode 710A, three characteristic stimulation amplitudes are represented respectively by markers 731A, 732A, and 733A along a direction 740A such as a line, a bar, or a rectangle projecting from the ring mode stimulation position 720A of the ring electrode 710A. Similarly, markers 731B, 732B, and 733B along a direction 740B represent characteristic stimulation amplitudes corresponding to the ring electrode 710B; markers 731C, 732C, and 733C along a direction 740C represent characteristic stimulation amplitudes corresponding to the ring electrode 710C; markers 731D, 732D, and 733D along a direction 740D represent characteristic stimulation amplitudes corresponding to the ring electrode 710D; markers 731E, 732E, and 733E along a direction 740E represent characteristic stimulation amplitudes corresponding to the ring electrode 710E.

The characteristic stimulation amplitudes each represent respective current amplitudes that, when applied to a specific electrode configuration, would result in respective clinical responses or clinical effects satisfying respective conditions. Such characteristic stimulation amplitudes can be searched or determined within the amplitude limit (Iₘₐₓ) that can be set by a user, such as approximately 10.5 mA as shown in FIG. 7. In the illustrated example, the first characteristic stimulation amplitude I₁ (any one of the markers 731A-731E), corresponds to a clinical response indicative of an initial improvement in patient symptom from a baseline. Such improvement can be assessed using a physiological signals (e.g., heart rate) or motor parameters (e.g., tremor, rigidity, bradykinesia). In an example, I₁ can be determined as the current amplitude corresponding to an initial improvement from baseline by a given amount. In another example, I₁ corresponds to first time the clinical response score reaches a specific level relative to (such as X% of) the current best clinical response score, where X% can be approximately 10-80% in an example.

The second characteristic stimulation amplitude I₂ (any one of markers 732A-732E) corresponds to a clinical response score exceeding a threshold. In an example, I₂ can be determined as the current amplitude corresponding to the highest clinical response score before a side effect has been detected or reported. In an example where multiple best clinical response scores exist (e.g., multiple current amplitudes that all result in clinical response scores exceeding a threshold), I₂ can be determined to be the lowest amplitude, or a median amplitude, among those multiple current amplitudes that result in the best clinical response scores. In some examples, instead of determining one current amplitude value, I₂ can be a range of current amplitudes with the corresponding clinical response scores each satisfying a specific criterion (e.g., exceeding a threshold). Accordingly, instead of being represented by a single marker at a point location (e.g., any one of marks 732A-732E), I₂ can be graphically represented as a bar, a segment, or a collection of segments, along the line projecting from the longitudinal position of the ring electrode (e.g., any one of 740A-740E).

The third characteristic stimulation amplitude I₃ (any one of markers 733A, 773D, and 733E) corresponds to a side effect being detected or reported by the patient, a loss of improvement of patient symptom from the baseline. In an example, I₃ can be determined as the current amplitude corresponding to the side effect being immediately below, or reaching a given amount (e.g., X%) from, an expected side effect. In an example, I₃ can be determined as the current amplitude corresponding to the therapeutic effect returning to pre-treatment baseline, within a given amount of the baseline, or outside given amount of the best clinical response score. When no side effect or loss of symptom improvement is reported when the stimulation current reaches the limit Iₘₐₓ, I₃ can be determined to be equivalent to Iₘₐₓ, as indicated by markers 773B and 733C.

In some example, any of characteristic stimulation amplitudes (e.g., I₁, I₂, and I₃) can be a minimum stimulation amplitude, within a specific amplitude range, that produces respective clinical response indicators or clinical effects satisfying respective conditions. In some examples, characteristic stimulation amplitudes can additionally or alternatively be determined for any "intermediate" longitudinal positions different than the positions of the physical electrodes on the lead (e.g., ring mode stimulation positions 720A-720E of the ring electrodes 710A-710E). In the illustrated example, for a ring mode stimulation position 720F located between the ring mode stimulation positions 720D and 720E, characteristic stimulation amplitudes (e.g., I₁, I₂, and I₃) can be determined based on the clinical response scores (including both the tested clinical response scores and the estimated clinical response scores) in the search space using similar methods for generating the characteristic stimulation amplitudes for the ring mode stimulation positions 720A-720E as discussed above. The characteristic stimulation amplitudes may be graphically displayed as markers 731F, 732F, and 733F linearly arranged along a direction 740F projecting from the ring mode stimulation position 720F.

In some examples, the processor 402 can determine a therapeutic window for an electrode level using the characteristic stimulation amplitudes for that electrode. The therapeutic window represents a range of stimulation amplitudes that can be programmed to the corresponding electrode without introducing substantial side effects. In an example, the therapeutic window can be defined between the first characteristic stimulation amplitude I₁ and the third characteristic stimulation amplitude I₃. In another example, the therapeutic window can be defined as a segment shorter than the range defined by I₁ and I₃ and covering the second characteristic stimulation amplitude I₂. The therapeutic window can be determined each of the ring electrodes, and graphically displayed on the diagram 700. FIG. 7 illustrates by way of example a therapeutic window 750A spans between the characteristic stimulation amplitudes represented by markers 731A and 733A and along the line projecting from the longitudinal position of the ring electrode 710A.

Although three characteristic stimulation amplitudes I₁, I₂, and I₃ are shown for each electrode configuration (e.g., a monopolar configuration involving a ring electrode), other numbers (e.g., two, four, or five) of characteristic stimulation amplitudes can be generated for one or more ring electrodes and displayed on the diagram 700. In some examples, characteristic stimulation amplitudes generated and displayed for one ring electrode can be different than the characteristic stimulation amplitudes generated and displayed for another ring electrode.

The diagram 700 may include electrode configurations directly tested within the search space, such as those configurations included in the first set of tested base stimulation settings (e.g., BBS₁ - BBS₄ as discussed above with reference to FIG. 5). Each of the directly tested electrode configurations has a corresponding stimulation amplitude value (x-axis) and electrode configuration represented by the longitudinal position of a central point of stimulation (CPS) on the lead. The directly tested electrode configurations can be graphically represented by respective markers on the diagram 700. In some examples, the directly tested electrode configurations can be differentiated by their respective clinical effects (e.g., therapeutic benefits versus side effects). For example, as illustrated in FIG. 7, the directly tested electrode configuration 772 is associated with clinical benefits without side effects, and located in the positive effect region 750. In contrast, another directly tested electrode configuration 774 is associated with side effects, and is located in the negative effect region 760. Electrode configurations in the positive effect region 750 (e.g., configuration 772) and those in the negative effect region 760 can be distinguishably displayed on the diagram 700, such as represented by different markers or colors.

FIG. 8 illustrates a 2D monopolar review diagram 800 of at least a portion of an electrode configuration and parameter search space comprising a plurality of segmented electrodes 810A-810C at different angular positions 820A-820C around a circumference of a directional lead 810 at a specific longitudinal position, where separate and non-identical stimulation currents are applied to respective segmented electrodes to deliver "directional mode stimulation". For each segmented electrode, multiple characteristic stimulation amplitudes, such as determined by the processor 402, can be graphically represented by markers linearly arranged along a direction such as a line, a bar, or a rectangle at an angle θ (relative to a reference direction) consistent with the angular position of the corresponding segmented electrode. The radius of the circular shape in diagram 800 represents a stimulation amplitude (e.g., current amplitude I in mA), up to an amplitude limit (Iₘₐₓ) that can be set by a user, applied to the monopolar configuration involving a segmented electrode. In the illustrated example, for segmented electrode 810A, three characteristic stimulation amplitudes can be represented respectively by markers 831A, 832A, and 833A along a direction 840A projecting from the lead center position 801 at an angle θ₁ based on the angular position 820A of the segmented electrode 810A. Similarly, for segmented electrode 810B, characteristic stimulation amplitudes are represented by markers 831B, 832B, and 833B along a direction 840B projecting from the lead center position 801 at an angle θ₂ based on the angular position 820B of the segmented electrode 810B. For segmented electrode 810C, characteristic stimulation amplitudes are represented by markers 831C, 832C, and 833C along a direction 840C projecting from the lead center position 801 at an angle θ₃ based on the angular position 820C of the segmented electrode 810C. Similar to the characteristic stimulation amplitudes I₁, I₂, and I₃ for the monopolar configurations involving respective ring mode stimulation electrodes as shown in FIG. 7, here in FIG. 8 the characteristic stimulation amplitudes can be searched or determined within the amplitude limit (Iₘₐₓ), represented by the radius of the circular shape, that can be set by a user. The characteristic stimulation amplitudes can include a first amplitude I₁ (any one of the markers 831A, 831B, or 831E) corresponding to a clinical response indicative of an initial improvement in patient symptom from a baseline, a second amplitude I₂ (any one of the markers 832A, 832B, or 832C) corresponding to a clinical response score exceeding a threshold, and a third amplitude I₃ (any one of the markers 833A, 833B, or 833C) corresponding to a side effect being detected or reported by the patient, or a loss of improvement of patient symptom from the baseline. In some examples, for each of the segmented electrodes 810A-810C, a therapeutic window can be defined between two characteristic stimulation amplitudes for that electrode, such as between the first amplitude I₁ and the third amplitude I₃. In some examples, in addition or alterative to the three characteristic stimulation amplitudes I₁, I₂, and I₃, other numbers (e.g., two, four, or five) of characteristic stimulation amplitudes can be generated for one or more segmented electrodes and displayed on the diagram 800. In some examples, characteristic stimulation amplitudes (e.g., I₁, I₂, and I₃) can be determined for "intermediate" angular positions different than the positions 820A-820C of the respective segmented electrodes 810A-810C on the lead. As similarly discussed above with reference to FIG. 7, the diagram 800 may include electrode configurations directly tested within the search space, such as those configurations included in the first set of tested base stimulation settings (e.g., BBS₁ - BBS₄ as discussed above with reference to FIG. 5). Such actually tested configurations may be distinguishably displayed based on their respective clinical effects (e.g., therapeutic benefits versus side effects). For example, electrode configurations in the positive effect region 850 and those in the negative effect region 860 can be distinguishably displayed on the diagram 800.

The characteristic stimulation amplitudes for each ring mode stimulation position (ring electrode or segmented electrode) as shown in FIGS. 7-8 represents stimulation amplitudes (e.g., current I) when other stimulation parameters, such as frequency (F) and pulse width (PW) as shown in FIG. 6, are fixed at receptive values (e.g., frequency of 130 Hz and PW of 60 µs). When the frequency (F) or pulse width (PW) are reprogrammed to different values, a different monopolar review diagram (similar to that shown in FIG. 7 or 8) can be generated, where the markers representing one or more of the characteristic stimulation amplitudes for a ring electrode or a segmented electrode can be repositioned along the line projecting from that ring electrode or segmented electrode.

In some examples, the 2D monopolar review diagram 700 for ring electrodes at different longitudinal levels of a lead (or for segmented electrodes of a given longitudinal level that are programmed to deliver "ring mode stimulation") may be combined with the 2D monopolar review 800 for segmented electrodes programmed to deliver "directional mode stimulation". In an example, the diagrams 700 and 800 may be stacked on top of each other. The combined monopolar review may include characteristic stimulation amplitudes (e.g., I₁, I₂, and I₃ as described above in reference to FIG. 7) for each ring electrode (or each set of segmented electrodes delivering ring mode stimulation), and characteristic stimulation amplitudes (e.g., I₁, I₂, and I₃ as described above in reference to FIG. 8) for each segmented electrode at a longitudinal level of the lead. The combined monopolar review may be especially helpful in better understanding clinical effects of electrostimulation using electrode configurations involving ring electrodes and segmented electrodes on a lead (e.g., lead 219).

The diagrams 700 or 800 may be displayed to a user, such as on the GUI 600 of the CP 129, where a user (e.g., a clinician or the patient) may select active electrodes or adjust stimulation parameter values within the " positive effect" region 661. For example, the user may only be allowed to select stimulation current amplitude for an electrode at a particular location to be within a specific amplitude range. In some examples, the diagrams 700 or 800 may be displayed on a user interface of the RC 128, such as a mobile device (e.g., a smartphone or a tablet) operable by the patient for in-home titration of an base stimulation program.

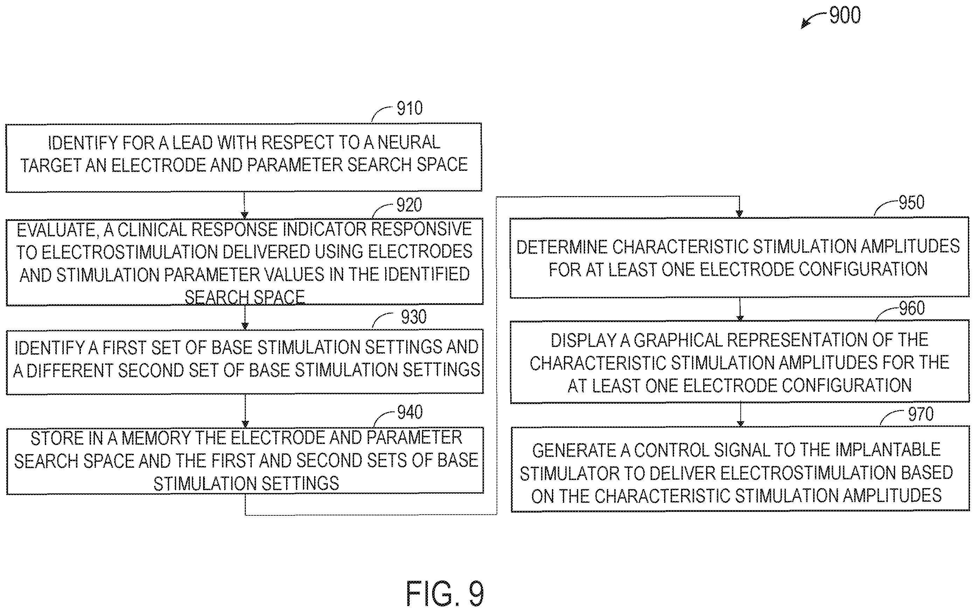

FIG. 9 is a flow chart illustrating, by way of example and not limitation, a non-claimed method 900 for generating and presenting electrostimulation data and patient clinical responses in a formatted report to guide electrostimulation therapy such as DBS. The method 900 may be carried out using a medical system such as the electrical stimulation system 100 in FIG. 1, or the electrical stimulation system 412 in FIG. 4. The electrostimulation may be generated by an implantable stimulator such as the IPG 127 or the IPG 210, and delivered to the neural target via a lead comprising a plurality of electrodes, such as the non-directional lead 218 or the directional lead 219. Portions of the method 900 may be implemented in an external device, such as the CP 129 or the RC 128 in FIG. 1 or the computing device 400 in FIG. 4.

The method 900 includes steps 910-940 for generating and storing in a memory one or more base stimulation settings as well as a search space of electrode configurations and parameter values; and steps 950-970 for determining and presenting characteristic stimulation amplitudes for one or more electrode configurations in formatted report. At 910, a search space for the lead with respect to the neural target can be identified, such as using the search space identifier 510. The search space can include a subset of the electrodes on the lead for delivering stimulation and stimulation parameter values or value ranges associated with the subset of electrodes. Identification of the search space can be based on spatial information of the lead, such as lead positions with respect to neural targets, which can be obtained from imaging data of the lead and patient anatomy. Additionally or alternatively, identification of the search space can be based on physiological information such as physiological signals sensed locally at electrode contacts. Certain limitations or constraints may be imposed on the electrode configurations and parameter values (e.g., current amplitude, frequency, or pulse width). In some examples, the search space may be graphically presented to a user (e.g., a clinician or a patient), such as a heatmap depicting intensities of clinical responses to stimulations delivered over a range of electrode positions and over a range of stimulation parameter values, as shown in FIGS. 7-8.

At 920, a clinical response indicator can be evaluated responsive to electrostimulation delivered using electrodes and stimulation parameter values taken from the identified search space. The evaluation of the clinical response indicator can be performed using a programming device such as the computing device 400, the CP 129 or the RC 128. Clinical responses may be evaluated for a plurality of electrodes at different locations and injected with respective stimulation current amplitudes. The clinical response may include therapeutic benefits and/or side effects on the patient. In an example, the clinical responses may be based on patient or clinician observations. For example, motor symptoms such as bradykinesia (slowness of movement), rigidity, tremor, among other symptoms or side effects, can be scored by the patient or by the clinician upon overserving or questioning the patient. In some examples, the clinical responses can be objective in nature, such as measurements automatically or semi-automatically taken by a sensor, such as a wearable sensor associated with a mobile device. The clinical responses may be converted to a clinical response score. In an example, a clinical score may be computed based on intensity, frequency, or duration of one or more of tremor, rigidity, or bradykinesia responses.

At 930, a first set of base stimulation settings and a different second set of base stimulation settings can be identified. Each base stimulation setting of the first and second sets comprises an electrode configuration and stimulation parameter values or value ranges selected from the search space. Each base stimulation setting may be identified based on the evaluation of the clinical response indicator. A base stimulation setting corresponds to a clinical response indicator satisfying a specific condition, such as the clinical response score falling within a specified range. A base stimulation setting, which includes an optimal electrode configuration and an optimal stimulation parameter value, can be determined using the feedback loop stimulation parameter control system 500, in which the electrode configuration or values of one or more stimulation parameters can be continuously or iteratively adjusted and the clinical response evaluated, until an optimal, desired, or acceptable outcome is reached, such as maximizing therapeutic efficacy while minimizing unwanted side effects, or until a specific stop condition is reached such as number of iterations, time spent in programming session, or the like.

Each base stimulation setting determined at 930 can be associated with a clinical response indicator. A base stimulation setting in association with the clinical response indicator is referred to as a stimulation program. Depending on how the clinical response score is computed, one or more optimal base stimulation settings may be determined. As illustrated in Table 1, base stimulation settings created and stored in the memory may include those base stimulation settings (e.g., BSS₁ - BSS₃) associated with the clinical response scores computed based on a single response effect (e.g., bradykinesia, tremor, or rigidity), or stimulation settings (e.g., BSS₄ - BSS₈) associated with clinical response scores computed using a weighted combination of two or more clinical effects, such as a%* bradykinesia + b%* tremor + c%* rigidity.

For a base stimulation setting of the first set, electrostimulation programmed with said base setting can be delivered to the patient, clinical effects (including therapeutic effects and/or side effects, or motor symptoms such as bradykinesia, tremor, or rigidity) may be detected, and a clinical response be evaluated based on the detected clinical effects. Because actual electrostimulation testing is administered, the first set of base stimulation settings are also referred to as "tested base stimulation settings", and the clinical response is referred to as "tested clinical response". Examples of the first set of base stimulation setting include BBS₁ - BBS₄ as shown in Table 1. In contrast, for a base stimulation setting of the second set, no electrostimuation is programmed or delivered to the patient. Instead, clinical effects may be predicted based at least on the clinical effects detected from the electrostimulation testing based on the first set of base stimulation settings, and a clinical response may be estimated using the predicted clinical effects. The prediction may be carried out using a trained prediction model, such as the machine learning engine 502 of the feedback control logic 501 in FIG. 5. Because no electrostimulation testing is administered, the second set of base stimulation settings are also referred to as "predicted" or "estimated" base stimulation settings, and the clinical response is referred to as "predicted" or "estimated" clinical response. Examples of the first set of base stimulation setting include BBS₅ - BBS₈ as shown in Table 1.

At 940, the search space identified from 910 and the first and second sets of base stimulations settings (along with respective clinical response indicators) identified from 930 can be stored in a memory, such as a memory internal to the IPG (e.g., IPG 127 or IPG 210). Upon request, one or more base stimulation settings and/or the search space may be retrieved from the memory.

At 950, one or more characteristic stimulation amplitudes can be determined for at least one electrode configuration, such as an electrode configuration of a base stimulation setting of the first set or the second set. The characteristic stimulation amplitudes can be determined based on the clinical response indicators of the first and second sets of base stimulation settings. The characteristic stimulation amplitudes each correspond to respective clinical response indicators or clinical effects satisfying respective conditions. By way of example and as illustrated in FIG. 7-8, the characteristic stimulation amplitudes may include one or more of a first characteristic stimulation amplitude I₁ that correspond to a clinical response indicative of an initial improvement in patient symptom from a baseline, a second characteristic stimulation amplitude I₂ that correspond to a clinical response score exceeding a threshold, a third characteristic stimulation amplitude I₃ that correspond to a side effect being detected or reported by the patient, or a loss of improvement of patient symptom from the baseline. In an example, characteristic stimulation amplitudes can be determined for each of a range of ring electrodes on a non-directional lead. In another example, characteristic stimulation amplitudes can be determined for a set of segmented electrodes around a circumference of the lead at a longitudinal position that are configured to deliver ring mode stimulation. In yet another example, characteristic stimulation amplitudes can be determined for each of a range of segmented electrodes on a directional lead that are configured to deliver directional mode stimulation.

At 960, a graphical representation of characteristic stimulation amplitudes for one or more electrode configurations, along with the clinical response data of the first and second sets of base stimulation settings, may be displayed on an output unit, such as the display 406. The graphical representation of characteristic stimulation amplitudes, along with other stimulation data (including electrode configurations, and search space with clinical responses measured or estimated over a range of electrode positions) can be formatted into a monopolar review report. The monopolar review report can be presented to a system user (e.g., a physician), as illustrated in FIGS. 7-8. Such monopolar review formatted report can help physicians better understand clinical effects of electrostimulations with a wide range of tested and untested electrode configurations and stimulation parameter values or value ranges. The formatted data report also facilitates consistent data reporting and data entry into other systems such as patient medical record system, thereby easing the burden and saving the cost of entry of such stimulation data.

At 970, a control signal can be generated and provided to the implantable stimulator to deliver electrostimulation based on the characteristic stimulation amplitudes for the at least one electrode configuration.

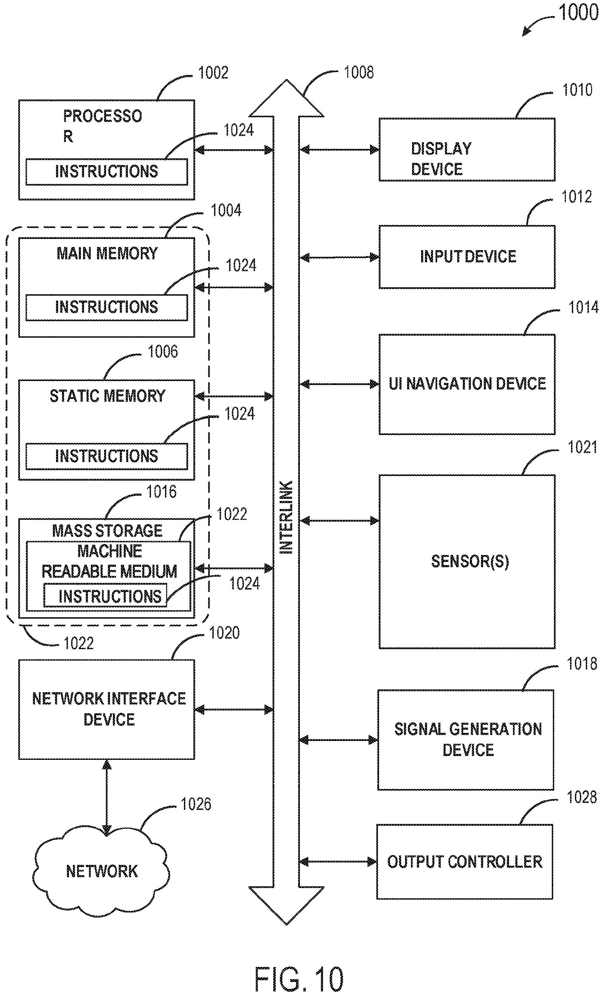

FIG. 10 illustrates generally a block diagram of an example machine 1000 upon which any one or more of the techniques (e.g., methodologies) discussed herein may perform. Portions of this description may apply to the computing framework of various portions of the neuromodulation device or the external programming device.

In alternative examples, the machine 1000 may operate as a standalone device or may be connected (e.g., networked) to other machines. In a networked deployment, the machine 1000 may operate in the capacity of a server machine, a client machine, or both in server-client network environments. In an example, the machine 1000 may act as a peer machine in peer-to-peer (P2P) (or other distributed) network environment. The machine 1000 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), among other computer cluster configurations.

Examples, as described herein, may include, or may operate by, logic or a number of components, or mechanisms. Circuit sets are a collection of circuits implemented in tangible entities that include hardware (e.g., simple circuits, gates, logic, etc.). Circuit set membership may be flexible over time and underlying hardware variability. Circuit sets include members that may, alone or in combination, perform specified operations when operating. In an example, hardware of the circuit set may be immutably designed to carry out a specific operation (e.g., hardwired). In an example, the hardware of the circuit set may include variably connected physical components (e.g., execution units, transistors, simple circuits, etc.) including a computer readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuit set in hardware via the variable connections to carry out portions of the specific operation when in operation. Accordingly, the computer readable medium is communicatively coupled to the other components of the circuit set member when the device is operating. In an example, any one of the physical components may be used in more than one member of more than one circuit set. For example, under operation, execution units may be used in a first circuit of a first circuit set at one point in time and reused by a second circuit in the first circuit set, or by a third circuit in a second circuit set at a different time.

Machine (e.g., computer system) 1000 may include a hardware processor 1002 (e.g., a central processing unit (CPU), a graphics processing unit (GPU), a hardware processor core, algorithm specific ASIC, or any combination thereof), a main memory 1004 and a static memory 1006, some or all of which may communicate with each other via an interlink (e.g., bus) 1008. The machine 1000 may further include a display unit 1010 (e.g., a raster display, vector display, holographic display, etc.), an alphanumeric input device 1012 (e.g., a keyboard), and a user interface (UI) navigation device 1014 (e.g., a mouse). In an example, the display unit 1010, input device 1012 and UI navigation device 1014 may be a touch screen display. The machine 1000 may additionally include a storage device (e.g., drive unit) 1016, a signal generation device 1018 (e.g., a speaker), a network interface device 1020, and one or more sensors 1021, such as a global positioning system (GPS) sensor, compass, accelerometer, or other sensors. The machine 1000 may include an output controller 1028, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The storage device 1016 may include a machine readable medium 1022 on which is stored one or more sets of data structures or instructions 1024 (e.g., software) embodying or utilized by any one or more of the techniques or functions described herein. The instructions 1024 may also reside, completely or at least partially, within the main memory 1004, within static memory 1006, or within the hardware processor 1002 during execution thereof by the machine 1000. In an example, one or any combination of the hardware processor 1002, the main memory 1004, the static memory 1006, or the storage device 1016 may constitute machine readable media.

While the machine-readable medium 1022 is illustrated as a single medium, the term "machine readable medium" may include a single medium or multiple media (e.g., a centralized or distributed database, and/or associated caches and servers) configured to store the one or more instructions 1024.

The term "machine readable medium" may include any medium that is capable of storing, encoding, or carrying instructions for execution by the machine 1000 and that cause the machine 1000 to perform any one or more of the techniques of the present disclosure, or that is capable of storing, encoding or carrying data structures used by or associated with such instructions. Nonlimiting machine-readable medium examples may include solid-state memories, and optical and magnetic media. In an example, a massed machine-readable medium comprises a machine readable medium with a plurality of particles having invariant (e.g., rest) mass. Accordingly, massed machine-readable media are not transitory propagating signals. Specific examples of massed machine-readable media may include: non-volatile memory, such as semiconductor memory devices (e.g., Electrically Programmable Read-Only Memory (EPROM), Electrically Erasable Programmable Read-Only Memory (EPSOM)) and flash memory devices; magnetic disks, such as internal hard disks and removable disks; magneto-optical disks; and CD-ROM and DVD-ROM disks.

The instructions 1024 may further be transmitted or received over a communication network 1026 using a transmission medium via the network interface device 1020 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as WiFi^{®}, IEEE 802.16 family of standards known as WiMax^{®}), IEEE 802.15.4 family of standards, peer-to-peer (P2P) networks, among others. In an example, the network interface device 1020 may include one or more physical jacks (e.g., Ethernet, coaxial, or phone jacks) or one or more antennas to connect to the communication network 1026. In an example, the network interface device 1020 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding or carrying instructions for execution by the machine 1000, and includes digital or analog communications signals or other intangible medium to facilitate communication of such software.

Various examples are illustrated in the figures above. One or more features from one or more of these examples may be combined to form other examples.

The method examples described herein may be machine or computer-implemented at least in part. Some examples may include a computer-readable medium or machine-readable medium encoded with instructions operable to configure an electronic device or system to perform methods as described in the above examples. An implementation of such methods may include code, such as microcode, assembly language code, a higher-level language code, or the like. Such code may include computer readable instructions for performing various methods. The code may form portions of computer program products. Further, the code may be tangibly stored on one or more volatile or non-volatile computer-readable media during execution or at other times.

The above detailed description is intended to be illustrative, and not restrictive. The scope of the disclosure should, therefore, be determined with references to the appended claims.

## Claims

1. A system (100) for providing electrostimulation to a patient comprising:
an implantable stimulator (127) configured to provide electrostimulation to a neural target of the patient via a lead (126) comprising a plurality of electrodes (132); and
a programming device (129) communicatively coupled to the implantable stimulator (127), the programming device (129) including a controller configured to:
identify a first set of base stimulation settings and a different second set of base stimulation settings, each base stimulation setting of the first and second sets comprising an electrode configuration and stimulation parameter values or value ranges selected from a search space of electrode configurations and parameter values for the lead with respect to the neural target;
for each base stimulation setting of the first set, detect one or more clinical effects in response to electrostimulation delivered according to the corresponding base stimulation setting, and evaluate a clinical response indicator using the detected one or more clinical effects;
for each base stimulation setting of the second set, without delivering electrostimulation, predict one or more clinical effects based at least on the detected clinical effects for the first set of base stimulation settings, and estimate a clinical response indicator using the predicted one or more clinical effects;
based on the clinical response indicators of the first and second sets of base stimulation settings, determine characteristic stimulation amplitudes for at least one electrode configuration in the search space, the characteristic stimulation amplitudes each corresponding to respective clinical response indicators satisfying respective conditions; and
display, on a user interface, a graphical representation of the characteristic stimulation amplitudes for the at least one electrode configuration,
wherein the characteristic stimulation amplitudes include one or more of:
a first stimulation amplitude corresponding to a clinical response indicator indicating an improvement in patient symptom from a baseline;
a second stimulation amplitude corresponding to a clinical response score exceeding a threshold, the clinical response score computed as a weighted combination of one or more clinical effects; or
a third stimulation amplitude corresponding to a clinical response indicator representing a side effect or a loss of improvement of patient symptom from the baseline.

2. The system of claim 1, wherein the characteristic stimulation amplitudes are determined for at least one of a monopolar electrode configuration in the first set of base stimulation settings, or a monopolar electrode configuration in the second set of base stimulation settings.

3. The system of claim 1 or 2, wherein the programming device is configured to generate a control signal to the implantable stimulator to deliver electrostimulation based on the characteristic stimulation amplitudes for the at least one electrode configuration.

4. The system of claim 1 to 3, wherein at least one of the first, the second, or the third stimulation amplitude is a minimum stimulation amplitude, within a specific amplitude range, with a corresponding clinical response indicator satisfying a specific condition.

5. The system of any of claims 1-4, wherein the programming device (129) further comprises a display screen for displaying a therapeutic window representing an amplitude range between the first characteristic stimulation amplitude and the third characteristic stimulation amplitude.

6. The system of any of claims 1-5, wherein the programming device (129) further comprises a display screen for displaying graphically the search space as a heatmap representing intensities of the clinical response indicators or clinical effects for the first set of base stimulation settings and the second set of base stimulation settings.

7. The system of claim 6, wherein the display screen is configured for displaying visually distinguishable indications of therapeutic effects and indications of side effects.

8. The system of any of claims 1-7, wherein the controller is configured to predict the one or more clinical effects for each of the second set of base stimulation settings, wherein the prediction includes applying the detected clinical effects for first set of base stimulation settings to a trained machine-learning model.

9. The system of any of claims 1-8, wherein the evaluated clinical response indicator includes a clinical response score computed using a weighted combination of the detected one or more clinical effects, and the estimated clinical response indicator includes a clinical response score computed using a weighted combination of the predicted one or more clinical effects.

10. The system of any of claims 1-9, wherein the one or more clinical effects include a therapeutic effect or a side effect of electrostimulation.

11. The system of any of claims 1-10, wherein the at least one electrode configuration includes one or more monopolar configurations each involving respective ring electrodes at different longitudinal positions on the lead, and
wherein the controller is configured to, for each of the one or more monopolar configurations involving a corresponding ring electrode, determine respective the characteristic stimulation amplitudes and graphically represent the respective characteristic stimulation amplitudes by markers linearly arranged in a direction with respect to the longitudinal position of the corresponding ring electrode.

12. The system of claim 11, wherein:
the characteristic stimulation amplitudes include (i) first characteristic stimulation amplitudes for a first monopolar configuration that includes a first ring electrode at a first longitudinal position on the lead, and (ii) second characteristic stimulation amplitudes for a second monopolar configuration that includes a second ring electrode at a second longitudinal position different than the first longitudinal position on the lead; and
the graphical representation of the characteristic stimulation amplitudes includes (i) first markers, representing the first characteristic stimulation amplitudes, that are linearly arranged along a first line projecting from the first longitudinal position, and (ii) second markers, representing the second characteristic stimulation amplitudes, that are linearly arranged along a second line projecting from the second longitudinal position and parallel to the first line.

13. The system of any of claims 1-12, wherein the at least one electrode configuration includes a monopolar configuration involving a set of segmented electrodes around a circumference of the lead at a longitudinal position, the set of segmented electrodes configured to deliver ring mode stimulation, and
wherein the controller is configured to, for the monopolar configuration involving the set of segmented electrodes configured to deliver ring mode stimulation, determine respective characteristic stimulation amplitudes by markers linearly arranged in a direction with respect to the longitudinal position of the set of segmented electrodes on the lead.

14. The system of any of claims 1-13, wherein the at least one electrode configuration includes one or more monopolar configurations each involving respective segmented electrodes at different angular positions around a circumference of the lead, and
wherein the controller is configured to, for the monopolar configurations involving a corresponding segmented electrode configured to deliver ring mode stimulation, determine respective characteristic stimulation amplitudes and graphically represent the characteristic stimulation amplitudes by markers linearly arranged in a direction with respect to the angular position of the corresponding segmented electrode.

15. The system of claim 14, wherein:
the characteristic stimulation amplitudes include (i) first characteristic stimulation amplitudes for a first monopolar configuration that includes a first segmented electrode at a first angular position around the circumference, and (ii) second characteristic stimulation amplitudes for a second monopolar configuration that includes a second segmented electrode at a second angular position different than the first angular position around the circumference; and
the graphical representation of the characteristic stimulation amplitudes includes (i) first markers, representing the first characteristic stimulation amplitudes, that are linearly arranged along a first line projecting from the first angular position, and (ii) second markers, representing the second characteristic stimulation amplitudes, that are linearly arranged along a second line projecting from the second angular position.

## Patentansprüche

1. System (100) zum Bereitstellen einer Elektrostimulation für einen Patienten, aufweisend:
einen implantierbaren Stimulator (127), der dafür konfiguriert ist, über eine Leitung (126) mit mehreren Elektroden (132) eine Elektrostimulation an einem neuronalen Ziel des Patienten bereitzustellen; und
eine Programmiervorrichtung (129), die mit dem implantierbaren Stimulator (127) kommunikativ verbunden ist, wobei die Programmiervorrichtung (129) eine Steuereinheit aufweist, die dafür konfiguriert ist:
einen ersten Satz von Basisstimulationseinstellungen und einen anderen zweiten Satz von Basisstimulationseinstellungen zu identifizieren, wobei jede Basisstimulationseinstellung des ersten und des zweiten Satzes eine Elektrodenkonfiguration und Stimulationsparameterwerte oder -wertebereiche aufweist, die aus einem Suchraum von Elektrodenkonfigurationen und Parameterwerten für die Leitung in Bezug auf das neuronale Ziel ausgewählt sind;
für jede Basisstimulationseinstellung des ersten Satzes eine oder mehrere klinische Wirkungen in Antwort auf eine gemäß der entsprechenden Basisstimulationseinstellung bereitgestellte Elektrostimulation zu erfassen und einen klinischen Antwortindikator unter Verwendung des einen oder der mehreren erfassten klinischen Wirkungen zu bewerten;
für jede Basisstimulationseinstellung des zweiten Satzes, ohne Bereitstellen einer Elektrostimulation, einen oder mehrere klinische Wirkungen auf der Grundlage zumindest der erfassten klinischen Wirkungen für den ersten Satz von Basisstimulationseinstellungen vorauszubestimmen und einen klinischen Antwortindikator unter Verwendung des vorausbestimmten einen oder der vorausbestimmten mehreren klinischen Wirkungen zu schätzen;
auf der Grundlage der klinischen Antwortindikatoren des ersten und des zweiten Satzes von Basisstimulationseinstellungen charakteristische Stimulationsamplituden für mindestens eine Elektrodenkonfiguration im Suchraum zu bestimmen, wobei die charakteristischen Stimulationsamplituden jeweils jeweiligen klinischen Antwortindikatoren entsprechen, die jeweilige Bedingungen erfüllen; und
auf einer Benutzeroberfläche eine grafische Darstellung der charakteristischen Stimulationsamplituden für die mindestens eine Elektrodenkonfiguration anzuzeigen,
wobei die charakteristischen Stimulationsamplituden eine oder mehrere der folgenden Amplituden aufweisen:
eine erste Stimulationsamplitude, die einem klinischen Antwortindikator entspricht, der eine Verbesserung der Patientensymptome gegenüber einer Basislinie anzeigt;
eine zweite Stimulationsamplitude, die einem klinischen Antwort-Score-Wert entspricht, der einen Schwellenwert überschreitet, wobei der klinische Antwort-Score-Wert als eine gewichtete Kombination aus einem oder mehreren klinischen Wirkungen berechnet wird; und
eine dritte Stimulationsamplitude, die einem klinischen Antwortindikator entspricht, der eine Nebenwirkung oder einen Verlust einer Verbesserung der Patientensymptome gegenüber der Basislinie darstellt.

2. System nach Anspruch 1, wobei die charakteristischen Stimulationsamplituden für eine monopolare Elektrodenkonfiguration im ersten Satz von Basisstimulationseinstellungen und/oder eine monopolare Elektrodenkonfiguration im zweiten Satz von Basisstimulationseinstellungen bestimmt werden.

3. System nach Anspruch 1 oder 2, wobei die Programmiervorrichtung dafür konfiguriert ist, ein Steuersignal für den implantierbaren Stimulator zu erzeugen, um eine Elektrostimulation basierend auf den charakteristischen Stimulationsamplituden für die mindestens eine Elektrodenkonfiguration bereitzustellen.

4. System nach einem der Ansprüche 1 bis 3, wobei mindestens eine unter der ersten, der zweiten und der dritten Stimulationsamplitude eine minimale Stimulationsamplitude innerhalb eines bestimmten Amplitudenbereichs ist, wobei ein entsprechender klinischer Antwortindikator eine bestimmte Bedingung erfüllt.

5. System nach einem der Ansprüche 1 bis 4, wobei die Programmiervorrichtung (129) ferner einen Bildschirm zum Anzeigen eines therapeutischen Fensters aufweist, das einen Amplitudenbereich zwischen der ersten charakteristischen Stimulationsamplitude und der dritten charakteristischen Stimulationsamplitude darstellt.

6. System nach einem der Ansprüche 1 bis 5, wobei die Programmiervorrichtung (129) ferner einen Bildschirm zum grafischen Anzeigen des Suchraums als Heatmap aufweist, die Intensitäten der klinischen Antwortindikatoren oder klinischen Wirkungen für den ersten Satz von Basisstimulationseinstellungen und den zweiten Satz von Basisstimulationseinstellungen darstellt.

7. System nach Anspruch 6, wobei der Bildschirm dafür konfiguriert ist, visuell unterscheidbare Anzeigen für therapeutische Wirkungen und Anzeigen für Nebenwirkungen darzustellen.

8. System nach einem der Ansprüche 1 bis 7, wobei die Steuereinheit dafür konfiguriert ist, die eine oder die mehreren klinischen Wirkungen für jede Basisstimulationseinstellung des zweiten Satzes von Basisstimulationseinstellungen durch Anwenden der erfassten klinischen Wirkungen für den ersten Satz von Basisstimulationseinstellungen auf ein trainiertes maschinelles Lernmodell vorauszubestimmen.

9. System nach einem der Ansprüche 1 bis 8, wobei der ausgewertete klinische Antwortindikator einen klinischen Antwort-Score-Wert aufweist, der unter Verwendung einer gewichteten Kombination der einen oder mehreren erfassten klinischen Wirkungen berechnet wird, und wobei der geschätzte klinische Antwortindikator einen klinischen Antwort-Score-Wert aufweist, der unter Verwendung einer gewichteten Kombination der einen oder der mehreren vorhergesagten klinischen Wirkungen berechnet wird.

10. System nach einem der Ansprüche 1 bis 9, wobei die eine oder die mehreren klinischen Wirkungen eine therapeutische Wirkung oder eine Nebenwirkung der Elektrostimulation aufweisen.

11. System nach einem der Ansprüche 1 bis 10, wobei die mindestens eine Elektrodenkonfiguration eine oder mehrere monopolare Konfigurationen aufweist, die jeweils entsprechende Ringelektroden an unterschiedlichen Längspositionen auf der Leitung aufweisen, und
wobei die Steuereinheit dafür konfiguriert ist, für jede unter der einen oder den mehreren monopolaren Konfigurationen, die eine entsprechende Ringelektrode aufweisen, die jeweiligen charakteristischen Stimulationsamplituden zu bestimmen und die jeweiligen charakteristischen Stimulationsamplituden durch Markierungen grafisch darzustellen, die linear in einer Richtung in Bezug auf die Längsposition der entsprechenden Ringelektrode angeordnet sind.

12. System nach Anspruch 11, wobei
die charakteristischen Stimulationsamplituden aufweisen:
(i) erste charakteristische Stimulationsamplituden für eine erste monopolare Konfiguration, die eine erste Ringelektrode an einer ersten Längsposition auf der Leitung aufweist; und
(ii) zweite charakteristische Stimulationsamplituden für eine zweite monopolare Konfiguration, die eine zweite Ringelektrode an einer zweiten Längsposition aufweist, die sich von der ersten Längsposition auf der Leitung unterscheidet, und
die grafische Darstellung der charakteristischen Stimulationsamplituden aufweist:
(i) erste Markierungen, die die ersten charakteristischen Stimulationsamplituden darstellen und linear entlang einer ersten Linie angeordnet sind, die von der ersten Längsposition hervorsteht; und
(ii) zweite Markierungen, die die zweiten charakteristischen Stimulationsamplituden darstellen und linear entlang einer zweiten Linie angeordnet sind, die von der zweiten Längsposition hervorsteht und parallel zur ersten Linie verläuft.

13. System nach einem der Ansprüche 1 bis 12, wobei die mindestens eine Elektrodenkonfiguration eine monopolare Konfiguration aufweist, die einen Satz von segmentierten Elektroden um einen Umfang der Leitung an einer Längsposition aufweist, wobei der Satz von segmentierten Elektroden dafür konfiguriert ist, eine Ringmodusstimulation bereitzustellen, und
wobei die Steuereinheit dafür konfiguriert ist, für die monopolare Konfiguration, die den Satz von segmentierten Elektroden aufweist, die dafür konfiguriert sind, eine Ringmodusstimulation bereitzustellen, jeweilige charakteristische Stimulationsamplituden durch Markierungen zu bestimmen, die linear in einer Richtung in Bezug auf die Längsposition des Satzes von segmentierten Elektroden auf der Leitung angeordnet sind.

14. System nach einem der Ansprüche 1 bis 13, wobei die mindestens eine Elektrodenkonfiguration eine oder mehrere monopolare Konfigurationen aufweist, die jeweils segmentierte Elektroden an unterschiedlichen Winkelpositionen um einen Umfang der Leitung herum aufweisen, und
wobei die Steuereinheit dafür konfiguriert ist, für die monopolaren Konfigurationen, die eine entsprechende segmentierte Elektrode aufweisen, die dafür konfiguriert ist, eine Ringmodusstimulation bereitzustellen, jeweilige charakteristische Stimulationsamplituden zu bestimmen und die charakteristischen Stimulationsamplituden durch Markierungen grafisch darzustellen, die linear in einer Richtung in Bezug auf die Winkelposition der entsprechenden segmentierten Elektrode angeordnet sind.

15. System nach Anspruch 14, wobei
die charakteristischen Stimulationsamplituden aufweisen:
(i) erste charakteristische Stimulationsamplituden für eine erste monopolare Konfiguration, die eine erste segmentierte Elektrode an einer ersten Winkelposition um den Umfang herum aufweist; und
(ii) zweite charakteristische Stimulationsamplituden für eine zweite monopolare Konfiguration, die eine zweite segmentierte Elektrode an einer zweiten Winkelposition aufweist, die sich von der ersten Winkelposition um den Umfang herum unterscheidet, und
die grafische Darstellung der charakteristischen Stimulationsamplituden aufweist:
(i) erste Markierungen, die die ersten charakteristischen Stimulationsamplituden darstellen und linear entlang einer ersten Linie angeordnet sind, die von der ersten Winkelposition hervorsteht; und
(ii) zweite Markierungen, die die zweiten charakteristischen Stimulationsamplituden darstellen und linear entlang einer zweiten Linie angeordnet sind, die von der zweiten Winkelposition hervorsteht.

## Revendications

1. Système (100) destiné à fournir une électrostimulation à un patient comprenant :
un stimulateur implantable (127) configuré pour fournir une électrostimulation à une cible neuronale du patient via un fil (126) comprenant une pluralité d'électrodes (132) ; et
un dispositif de programmation (129) couplé en communication avec le stimulateur implantable (127), le dispositif de programmation (129) incluant un dispositif de commande configuré pour :
identifier un premier ensemble de réglages de stimulation de base et un deuxième ensemble différent de réglages de stimulation de base, chaque réglage de stimulation de base des premier et deuxième ensembles comprenant une configuration d'électrodes et des valeurs ou des plages de valeurs de paramètre de stimulation sélectionnées depuis un espace de recherche de configurations d'électrodes et de valeurs de paramètre pour le fil par rapport à la cible neuronale ;
pour chaque réglage de stimulation de base du premier ensemble, détecter un ou plusieurs effets cliniques en réponse à une électrostimulation émise en fonction du réglage de stimulation de base correspondant, et évaluer un indicateur de réponse clinique à l'aide des un ou plusieurs effets cliniques détectés ;
pour chaque réglage de stimulation de base du deuxième ensemble, sans émission d'électrostimulation, anticiper un ou plusieurs effets cliniques sur la base au moins des effets cliniques détectés pour le premier ensemble de réglages de stimulation de base, et estimer un indicateur de réponse clinique à l'aide des un ou plusieurs effets cliniques anticipés ;
sur la base des indicateurs de réponse clinique des premier et deuxième ensembles de réglages de stimulation de base, déterminer des amplitudes de stimulation caractéristiques pour au moins une configuration d'électrodes dans l'espace de recherche, les amplitudes de stimulation caractéristiques correspondant chacune à des indicateurs de réponse clinique respectifs satisfaisant des conditions respectives ; et
afficher, sur une interface utilisateur, une représentation graphique des amplitudes de stimulation caractéristiques pour la au moins une configuration d'électrodes,
dans lequel les amplitudes de stimulation caractéristiques incluent une ou plusieurs parmi :
une première amplitude de stimulation correspondant à un indicateur de réponse clinique indiquant une amélioration dans un symptôme de patient à partir d'une base de référence ;
une deuxième amplitude de stimulation correspondant à un score de réponse clinique dépassant un seuil, le score de réponse clinique calculé en tant que combinaison pondérée d'un ou plusieurs effets cliniques ; ou
une troisième amplitude de stimulation correspondant à un indicateur de réponse clinique représentant un effet secondaire ou une perte d'amélioration d'un symptôme de patient à partir de la base de référence.

2. Système selon la revendication 1, dans lequel les amplitudes de stimulation caractéristiques sont déterminées pour au moins l'une parmi une configuration d'électrodes monopolaire dans le premier ensemble de réglages de stimulation de base ou une configuration d'électrodes monopolaire dans le deuxième ensemble de réglages de stimulation de base.

3. Système selon la revendication 1 ou 2, dans lequel le dispositif de programmation est configuré pour produire un signal de commande vers le stimulateur implantable afin d'émettre une électrostimulation sur la base des amplitudes de stimulation caractéristiques pour la au moins une configuration d'électrodes.

4. Système selon la revendication 1 à 3, dans lequel au moins l'une parmi la première, la deuxième ou la troisième amplitude de stimulation est une amplitude de stimulation minimale, à l'intérieur d'une plage d'amplitude spécifique, avec un indicateur de réponse clinique correspondant satisfaisant une condition spécifique.

5. Système selon l'une quelconque des revendications 1-4, dans lequel le dispositif de programmation (129) comprend en outre un écran d'affichage pour afficher une fenêtre thérapeutique représentant une plage d'amplitude entre la première amplitude de stimulation caractéristique et la troisième amplitude de stimulation caractéristique.

6. Système selon l'une quelconque des revendications 1-5, dans lequel le dispositif de programmation (129) comprend en outre un écran d'affichage pour afficher graphiquement l'espace de recherche en tant que carte de densité représentant des intensités des indicateurs de réponse clinique ou des effets cliniques pour le premier ensemble de réglages de stimulation de base et le deuxième ensemble de réglages de stimulation de base.

7. Système selon la revendication 6, dans lequel l'écran d'affichage est configuré pour afficher de façon à pouvoir distinguer visuellement des indications d'effets thérapeutiques et des indications d'effets secondaires.

8. Système selon l'une quelconque des revendications 1-7, dans lequel le dispositif de commande est configuré pour anticiper les un ou plusieurs effets cliniques pour chacun parmi le deuxième ensemble de réglages de stimulation de base inclut l'application des effets cliniques détectés pour un premier ensemble de réglages de stimulation de base à un modèle d'apprentissage machine entraîné.

9. Système selon l'une quelconque des revendications 1-8, dans lequel l'indicateur de réponse clinique évalué inclut un score de réponse clinique calculé à l'aide d'une combinaison pondérée des un ou plusieurs effets cliniques détectés, et l'indicateur de réponse clinique estimé inclut un score de réponse clinique calculé à l'aide d'une combinaison pondérée des un ou plusieurs effets cliniques anticipés.

10. Système selon l'une quelconque des revendications 1-9, dans lequel les un ou plusieurs effets cliniques incluent un effet thérapeutique ou un effet secondaire d'électrostimulation.

11. Système selon l'une quelconque des revendications 1-10, dans lequel la au moins une configuration d'électrodes inclut une ou plusieurs configurations monopolaires impliquant chacune des électrodes annulaires respectives en des positions longitudinales différentes sur le fil, et
dans lequel le dispositif de commande est configuré pour, pour chacune des une ou plusieurs configurations monopolaires impliquant une électrode annulaire correspondante, déterminer respectives les amplitudes de stimulation caractéristiques sont graphiquement représentent les amplitudes de stimulation caractéristiques respectives par des marqueurs agencés de façon linéaire dans une direction par rapport à la position longitudinale de l'électrode annulaire correspondante.

12. Système selon la revendication 11, dans lequel :
les amplitudes de stimulation caractéristiques inclut (i) des premières amplitudes de stimulation caractéristiques pour une première configuration monopolaire qui inclut une première électrode annulaire en une première position longitudinale sur le fil, et (ii) des deuxièmes amplitudes de stimulation caractéristiques pour une deuxième configuration monopolaire qui inclut une deuxième électrode annulaire en une deuxième position longitudinale différente de la première position longitudinale sur le fil ; et
la représentation graphique des amplitudes de stimulation caractéristiques inclut (i) des premiers marqueurs, représentant les premières amplitudes de stimulation caractéristiques, qui sont agencés de façon linéaire le long d'une première ligne se projetant depuis la première position longitudinale, et (ii) des deuxièmes marqueurs, représentant les deuxièmes amplitudes de stimulation caractéristiques, qui sont agencés de façon linéaire le long d'une deuxième ligne se projetant depuis la deuxième position longitudinale et en parallèle à la première ligne.

13. Système selon l'une quelconque des revendications 1-12, dans lequel la au moins une configuration d'électrodes inclut une configuration monopolaire impliquant un ensemble d'électrodes segmentées autour d'une circonférence du fil en une position longitudinale, l'ensemble d'électrodes segmentées configuré pour émettre une stimulation de mode annulaire, et
dans lequel le dispositif de commande est configuré pour, pour la configuration monopolaire impliquant l'ensemble d'électrodes segmentées configuré pour émettre une stimulation de mode annulaire, déterminer des amplitudes de stimulation caractéristiques respectives par des marqueurs agencés de façon linéaire dans une direction par rapport à la position longitudinale de l'ensemble d'électrodes segmentées sur le fil.

14. Système selon l'une quelconque des revendications 1-13, dans lequel la au moins une configuration d'électrodes inclut une ou plusieurs configurations monopolaires impliquant chacune des électrodes segmentées respectives en des positions angulaires différentes autour d'une circonférence du fil, et
dans lequel le dispositif de commande est configuré pour, pour les configurations monopolaires impliquant une électrode segmentée correspondante configurée pour émettre une stimulation de mode annulaire, déterminer des amplitudes de stimulation caractéristiques respectives et représenter graphiquement les amplitudes de stimulation caractéristiques par des marqueurs agencés de façon linéaire dans une direction par rapport à la position angulaire de l'électrode segmentée correspondante.

15. Système selon la revendication 14, dans lequel :
les amplitudes de stimulation caractéristiques incluent (i) des premières amplitudes de stimulation caractéristiques pour une première configuration monopolaire qui inclut une première électrode segmentée en une première position angulaire autour de la circonférence, et (ii) des deuxièmes amplitudes de stimulation caractéristiques pour une deuxième configuration monopolaire qui inclut une deuxième électrode segmentée en une deuxième position angulaire différente de la première position angulaire autour de la circonférence ; et
la représentation graphique des amplitudes de stimulation caractéristiques inclut (i) des premiers marqueurs, représentant les premières amplitudes de stimulation caractéristiques, qui sont agencés de façon linéaire le long d'une première ligne se projetant depuis la première position angulaire, et (ii) des deuxièmes marqueurs, représentant les deuxièmes amplitudes de stimulation caractéristiques, qui sont agencés de façon linéaire le long d'une deuxième ligne se projetant depuis la deuxième position angulaire.
